# EUROPEAN PATENT APPLICATION

(11) **EP 4 516 349 A1**
(43) Date of publication of application: **05.03.2025**
(21) Application number: 22944561.4
(22) Date of filing: 29.09.2022
(51) Int. Cl.: A61N 5/10

(54) **QUALITY GUARANTEE METHOD AND SYSTEM**

(30) Priority: 31.05.2022 CN 202210611450
(71) Applicant: Shanghai United Imaging Healthcare Co., Ltd., Shanghai 201807 (CN)
(72) Inventor: LIU, Yanfang, Shanghai 201807 (CN)
(74) Representative: Wang, Bo
(86) International application number: PCT/CN2022/122413
(87) International publication number: WO 2023/231253

(57) **Abstract**

The embodiments of the present disclosure provide a quality assurance method and system. The quality assurance method comprises: obtaining status information of a medical device; obtaining a target plan of a target subject; determining a prediction result based on the status information of the medical device and the target plan; and determining whether a quality assurance test is passed based on the prediction result.

## Description

### CROSS-REFERENCE TO RELATED APPLICATIONS

This application claims priority to Chinese Patent Application No. 202210611450.9, filed on May 31, 2022, the entire contents of which are incorporated herein by reference.

### TECHNICAL FIELD

The present disclosure generally relates to a radiotherapy system and method, and in particular, to a quality assurance system and method in radiotherapy.

### BACKGROUND

Radiotherapy is a very important treatment method in the treatment of tumors. To ensure the accurate implementation of the radiotherapy plan, a quality assurance test needs to be performed on the radiotherapy plan of the patient. However, the workflow of the quality assurance test of the radiotherapy plan is time-consuming, the third-party equipment for the quality assurance test is usually expensive, the use process is cumbersome, and when the radiotherapy plan fails, it is difficult to analyze the reasons why the radiotherapy plan fails. Therefore, it is desirable to provide a quality assurance system and method to improve the efficiency of the quality assurance test.

### SUMMARY

One of the embodiments of the present disclosure provides a quality assurance method. The quality assurance method may comprise: obtaining status information of a medical device; obtaining a target plan of a target subject; determining a prediction result based on the status information of the medical device and the target plan; and determining whether a quality assurance test is passed based on the prediction result.

In some embodiments, the status information of the medical device may include at least one of beam information corresponding to a plurality of dose rates, positioning accuracy information of at least one component of the medical device corresponding to a plurality of positions, or an operation error of at least one component of the medical device.

In some embodiments, the obtaining status information of a medical device may include: obtaining at least one data set, the at least one data set being determined based on at least one of a candidate plan, a limit performance of the at least one component of the medical device, or a calculation limit of a dose model; and determining the status information of the medical device by implementing the at least one data set using the medical device.

In some embodiments, the determining a prediction result based on the status information of the medical device and the target plan may include: determining whether the status information of the medical device satisfies a preset condition; and in response to determining that the status information of the medical device satisfies the preset condition, determining the prediction result based on the status information of the medical device and the target plan.

In some embodiments, the at least one data set may include at least one of a first data set, a second data set, or a third data set. The obtaining at least one data set may include: determining the first data set based on at least one first candidate plan, a value of a first candidate parameter of the at least one first candidate plan being within a first preset range; determining the second data set based on at least one second candidate plan, a value of a second candidate parameter of the at least one second candidate plan being outside a second preset range; and determining the third data set based on the limit performance of the at least one component of the medical device and/or the calculation limit of the dose model.

In some embodiments, the at least one data set may be the second data set or the third data set. The determining whether the status information of the medical device satisfies a preset condition may include: determining a plurality of test results by performing a plurality of tests based on the at least one data set; and determining whether the status information of the medical device satisfies the preset condition based on the plurality of test results.

In some embodiments, the obtaining a target plan of a target subject may include: determining a feature associated with a complexity of the target plan and/or a target flux map based on the target plan.

In some embodiments, the determining a prediction result based on the status information of the medical device and the target plan may include: determining the prediction result based on the status information of the medical device and the target plan of the target subject using a quality assurance model, the quality assurance model being a machine learning model.

In some embodiments, the quality assurance model may include a first model. The determining the prediction result based on the status information of the medical device and the target plan of the target subject using a quality assurance model may include: determining the prediction result by inputting the status information of the medical device and the target plan of the target subject into the first model, the prediction result including at least one of a predicted image, a gamma pass rate, or a dose distribution.

In some embodiments, the quality assurance model may include a second model. The quality assurance method may further comprise: in response to determining that the quality assurance test is not passed, determining a reason why the quality assurance test is not passed based on the status information of the medical device, the target plan of the target subject, and the prediction result using the second model.

In some embodiments, the quality assurance method may further comprise: obtaining an adjusted parameter value by adjusting a parameter value based on the reason why the quality assurance test is not passed; determining an updated prediction result based on the adjusted parameter value using the first model; determining whether the quality assurance test is passed based on the updated prediction result; and in response to determining that the quality assurance test is passed, controlling the medical device to implement an updated target plan on the target subject, the updated target plan being determined based on the adjusted parameter value.

In some embodiments, the target subject may include a plurality of regions of interest (ROI). The prediction result may include dose distributions corresponding to the plurality of ROIs. The determining whether the quality assurance test is passed based on the prediction result may include: obtaining a weight value corresponding to each of the plurality of ROIs; and determining whether the quality assurance test is passed based on the weight value corresponding to each of the plurality of ROIs and the dose distributions corresponding to the plurality of ROIs.

In some embodiments, the determining the prediction result based on the status information of the medical device and the target plan may include: obtaining an image of the target subject; and determining a three-dimensional dose distribution in the target subject based on the status information of the medical device, the target plan of the target subject, and the image of the target subject using the quality assurance model.

One of the embodiments of the present disclosure provides a quality assurance system. The quality assurance system may comprise: at least one storage device configured to store an instruction set; and at least one processor in communication with the at least one storage device. When executing the instruction set, the at least one processor may be configured to cause the system to: obtain status information of a medical device; obtain a target plan of a target subject; determine a prediction result based on the status information of the medical device and the target plan; and determine whether a quality assurance test is passed based on the prediction result.

One of the embodiments of the present disclosure provides a non-transitory computer-readable medium. The non-transitory computer-readable medium may comprise at least one set of instructions that, when executed by at least one processor of a computing device, may cause the at least one processor to implement a method, comprising: obtaining status information of a medical device; obtaining a target plan of a target subject; determining a prediction result based on the status information of the medical device and the target plan; and determining whether a quality assurance test is passed based on the prediction result

One of the embodiments of the present disclosure provides a quality assurance system. The quality assurance system may comprise a first obtaining module, configured to obtain status information of a medical device; a second obtaining module, configured to obtain a target plan of a target subject; and a determination module, configured to determine a prediction result based on the status information of the medical device and the target plan; and determine whether a quality assurance test is passed based on the prediction result.

One of the embodiments of the present disclosure provides a quality assurance device. The quality assurance device may be configured to implement the quality assurance method described above.

Some additional features of the present disclosure may be explained in the following description. Some additional features of the present disclosure will be apparent to those skilled in the art through the study of the following description and the corresponding drawings or understanding of the production or operation of the embodiments. The features of the present disclosure may be realized and achieved through practice or use of the methods, means, and combinations of various aspects of the specific embodiments described below.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, where:
FIG. 1 is a schematic diagram illustrating an exemplary medical system according to some embodiments of the present disclosure;
FIG. 2 is a schematic diagram illustrating an exemplary computing device on which a processing device is implemented according to some embodiments of the present disclosure;
FIG. 3 is a schematic diagram illustrating exemplary hardware and/or software components of a mobile device on which a terminal is implemented according to some embodiments of the present disclosure;
FIG. 4 is a block diagram illustrating an exemplary processing device according to some embodiments of the present disclosure;
FIG. 5 is a flowchart illustrating an exemplary quality assurance process according to some embodiments of the present disclosure;
FIG. 6 is a flowchart illustrating an exemplary quality assurance process according to some embodiments of the present disclosure; and
FIG. 7 is a flowchart illustrating an exemplary process for joint training of a first model and a second model according to some embodiments of the present disclosure.

### DETAILED DESCRIPTION

To more clearly illustrate the technical solutions of the embodiments of the present disclosure, the drawings required for use in the description of the embodiments will be briefly introduced below. However, it should be understood by those skilled in the art that the present disclosure can be implemented without these details. In other cases, to avoid unnecessarily obscuring various aspects of the present disclosure, well-known methods, processes, systems, components, and/or circuits have been described at a higher level. For those of ordinary skill in the art, it is obvious that various changes can be made to the disclosed embodiments, and the general principles defined in the present disclosure can be applied to other embodiments and application scenarios without departing from the principles and scope of the present disclosure. Therefore, the present disclosure is not limited to the embodiments shown, but conforms to the broadest scope consistent with the scope of the patent application.

The terms used in the present disclosure are only for the purpose of describing specific example embodiments and are not restrictive. The singular forms "a," "an," and "the" used in the present disclosure may also include plural forms unless the context clearly indicates an exception. It should also be understood that the terms "including" and "comprising" used in the present disclosure only indicate the presence of the features, steps, operations, components, and/or parts, but do not exclude the presence or addition of other features, steps, operations, components, parts, and/or combinations thereof.

It should be understood that the terms "system," "engine," "device," "unit," "module," and/or "block" used herein are a way to distinguish between different components, elements, parts, sections, or assemblies at different levels. However, the terms may be replaced by other expressions if other words accomplish the same purpose.

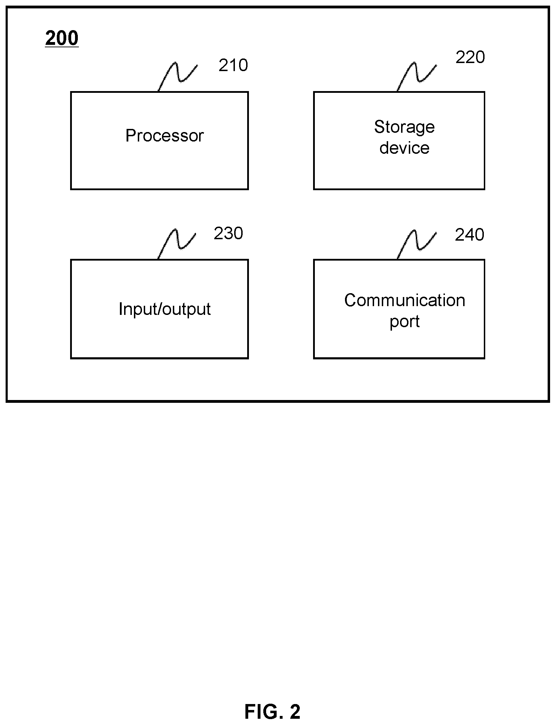

Generally, the words "module," "unit," or "block" used in the present disclosure refer to logic embodied in hardware or firmware, or a collection of software instructions. The module, unit, or block described in the present disclosure may be implemented as software and/or hardware, and may be stored in any type of non-transitory computer-readable medium or other storage device. In some embodiments, software modules/units/blocks may be compiled and linked to an executable program. It is appreciated that software modules may be callable from other modules/units/blocks or themselves, and/or may be called in response to detected events or interrupts. Software modules/units/blocks configured to be executed on a computing device (e.g., a processor 210 as shown in FIG. 2) may be provided on a computer-readable medium, such as an optical disc, a digital video disc, a flash drive, a disk, or any other tangible medium, or as a digital download (and may be initially stored in a compressed or installable format, requiring installation, decompression or decryption prior to execution). The software code here may be partially or completely stored in the storage device of the computing device that performs the operation, and applied in the operation of the computing device. Software instructions may be embedded in firmware such as an EPROM. It should also be understood that the hardware modules/units/blocks may be included in connected logical components, such as gates and triggers, and/or may include programmable units, such as programmable gate arrays or processors. The modules/units/blocks or computing device functions described in the present disclosure may be implemented as software modules/units/blocks, but may be represented in hardware or firmware. Typically, the modules/units/ blocks described herein refer to logic modules/units/blocks, which may be combined with other modules/units/blocks or divided into submodules/subunits/subblocks, although they are physical organizations or storage devices. This description may be applicable to a system, an engine, or a portion thereof.

It is understood that, unless the context clearly indicates otherwise, when a unit, engine, module, or block is referred to as being "on," "connected," or "coupled to" another unit, engine, module, or block, it may be directly on, connected or coupled to or in communication with the other unit, engine, module, or block, or there may be intermediate units, engines, modules or blocks. In the present disclosure, the term "and/or" may include any one or more of the relevant listed items or combinations thereof.

The present disclosure may become more apparent from the following description of the accompanying drawings, which form a part of the present disclosure. However, it should be understood that the drawings are for illustration and description purposes only and are not intended to limit the scope of the present disclosure. It should be understood that the drawings are not drawn to scale.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, operations can be processed in reverse order or simultaneously. Also, it is possible to add other operations to these processes or to remove an operation or operations from these processes.

Radiotherapy refers to treating tumors using radiation. Due to the high energy of the beam, normal cells are affected while tumor cells are killed. To minimize the damage to normal tissues, a radiotherapy plan needs to be formulated. And to ensure that the radiotherapy plan can be accurately implemented, a quality assurance test needs to be performed on the radiotherapy plan. The quality assurance test of the radiotherapy plan refers to the detection of parameters (e.g., a dose distribution) involved in the process of the radiotherapy plan of a patient.

In the current quality assurance test of treatment plans, a measured dose distribution is obtained using a physical device (e.g., a dose film, an ionization chamber, an electronic portal imaging device (EPID)), and the measured dose distribution is compared with a calculated dose distribution determined by a radiotherapy planning system (TPS). A gamma pass rate is determined through gamma analysis to determine whether the quality assurance test is passed. However, this method is usually expensive and time-consuming, and requires independent verification of the radiotherapy plan of each patient, and it is difficult to determine the reason why the quality assurance test is not passed. In addition, in some online radiotherapy processes (e.g., an online adaptive radiotherapy), there are usually no actual test conditions for the quality assurance test.

The present disclosure provides a quality assurance method and system. Specifically, the quality assurance system may obtain status information (e.g., beam information corresponding to a plurality of dose rates, positioning accuracy information of at least one component of a medical device corresponding to a plurality of positions, an operation error of at least one component of the medical device, and a test result of the medical device executing a data set) of the medical device. The quality assurance system may obtain a target plan (e.g., a feature related to the complexity of the target plan, and a target flux map) of a target subject. The quality assurance system may determine a prediction result (e.g., a predicted EPID image, the gamma pass rate, the dose distribution, the reasons why the quality assurance test is not passed, and a target adjustment mode) based on the status information of the medical device and the target plan. For example, the status information of the medical device, the target plan of the target subject, and/or parameter information of a dose model may be input into a quality assurance model, and the quality assurance model may output the prediction result. The quality assurance system may determine whether the quality assurance test is passed based on the prediction result. In response to determining that the quality assurance test is passed, the quality assurance system may control the medical device to perform a target operation (e.g., a radiotherapy operation) on the target subject. Since the influence of multiple types of information such as the medical device, the target plan, and the dose model on the prediction result is considered at the same time, the rationality, accuracy, and effectiveness of the quality assurance test can be improved. When the prediction result satisfies a preset condition, it can be determined that the quality assurance test is passed, and then the medical device can be controlled to perform the radiotherapy operation on the target subject based on the target plan without performing the quality assurance test on the target plan using the medical device, thereby simplifying the radiotherapy process. In addition, the quality assurance system can also analyze the reason why the quality assurance test is not passed based on the prediction result, and further provide recommended adjustments, so as to improve the efficiency of the radiotherapy process.

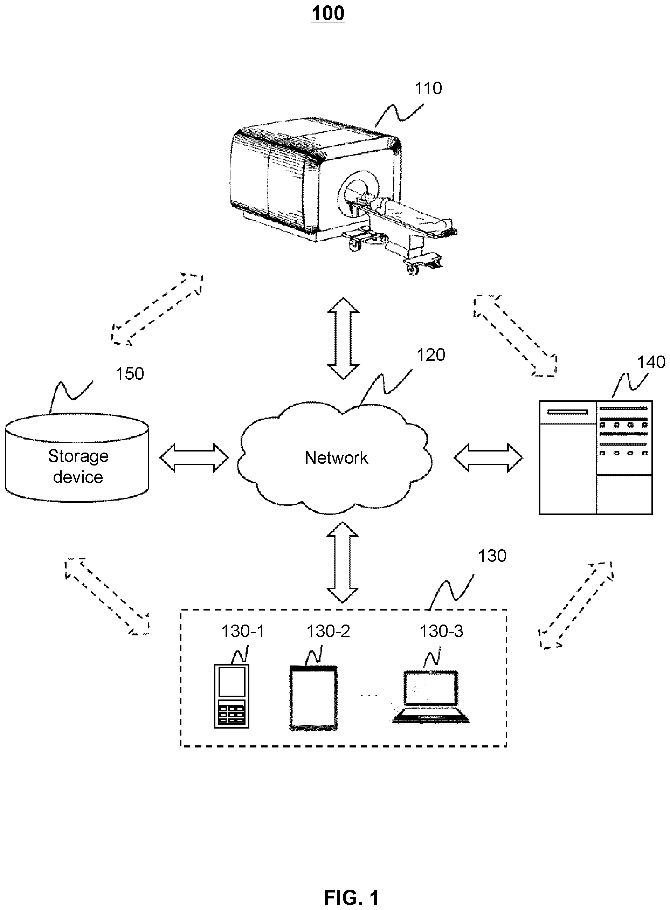

FIG. 1 is a schematic diagram illustrating an exemplary medical system according to some embodiments of the present disclosure. A medical system 100 may include a medical device 110, a network 120, one or more terminals 130, a processing device 140, and a storage device 150. One or more components of the medical system 100 may be connected in various ways. Merely by way of example, the medical device 110 may be connected with the processing device 140 directly (as shown by dotted double-headed arrows connecting the medical device 110 and the processing device 140) or via the network 120. As another example, the storage device 150 may be connected with the medical device 110 directly (as shown by dotted double-headed arrows connecting the storage device 150 and the medical device 110) or via the network 120. As another example, the one or more terminals 130 may be connected with the processing device 140 directly (as shown by dotted double-headed arrows connecting the terminal 130 and the processing device 140) or via the network 120. As another example, the one or more terminals 130 may be connected with storage device 150 directly (as shown by dotted double-headed arrows connecting the one or more terminals 130 and the storage device 150) or via network 120.

The medical device 110 may be configured to image and/or treat a subject (e.g., a target subject). In some embodiments, the subject may include a biological subject and/or a non-biological subject. For example, the subject may include a specific part of a human body, such as the head, the chest, the abdomen, etc. As another example, the subject may be a patient to be scanned by the medical device 110.

In some embodiments, the medical device 110 may be a medical treatment device for disease treatment or research purposes. In some embodiments, the medical device 110 may include a single-modality device, such as an X-ray therapy device, a Co-60 teletherapy device, a medical electron accelerator, etc. In some embodiments, the medical device 110 may be a multi-modality (e.g., dual-modality) device to obtain a medical image related to the subject and perform radiotherapy on the subject. For example, the medical device 110 may include an image-guided radiation therapy (IGRT) device, such as a CT-guided radiation therapy device, an MRI-guided radiation therapy device. The devices provided above are for illustrative purposes only and are not intended to limit the scope of the present disclosure. As used in the present disclosure, the term "imaging modality" or "modality" refers to an imaging process or technique for collecting, generating, processing, and/or analyzing imaging information of the subject.

In some embodiments, the medical device 110 may include a radiotherapy component, such as a treatment head. The treatment head may be connected with a gantry. In some embodiments, the treatment head may move with a movement (e.g., rotation) of the gantry. The treatment head may include a target, a therapeutic radiation source, and a beam limiter. The therapeutic radiation source may be configured to emit a radiation beam to the subject. In some embodiments, the therapeutic radiation source may be a radiation source for emitting a beam in a megavolt energy range (i.e., the energy of the beam is greater than 1 megavolt), or a radiation source for emitting a beam in a kilovolt energy range (i.e., the energy of the beam is greater than 1 kilovolt). The beam limiter may include a beam limiter hole, a primary collimator, and a secondary collimator. The secondary collimator may include a multi-leaf collimator, a tungsten gate, etc. The multi-leaf collimator may include a plurality of leaves. In some embodiments, the positions of the plurality of leaves of the multi-leaf collimator and/ or the tungsten gate may be configured to form a radiation region, i.e., a radiation field. In some embodiments, the plurality of leaves may be driven by one or more drive components (e.g., a motor) to move to a specific position to change the shape of the radiation field.

In some embodiments, the medical device 110 may include a radiotherapy auxiliary device, such as an electronic portal imaging device (EPID). The EPID may be configured to generate an image of the subject before, during, and/or after the treatment. The EPID may include a detector for detecting radiation (e.g., X-rays, and gamma rays) emitted from the therapeutic radiation source. In some embodiments, the detector may include one or more detection units. The one or more detection units may include a scintillation detector (e.g., a cesium iodide detector, and a gadolinium oxysulfide detector), a gas detector, etc. The one or more detection units may include a single row of detectors or multiple rows of detectors.

In some embodiments, the medical device 110 may also include an imaging device. The imaging device may include one of a computer tomography (CT) device, an ultrasound imaging component, a fluoroscopic imaging component, a magnetic resonance imaging (MRI) device, a single photon emission computed tomography (SPECT) device, a positron emission tomography (PET) device, or any combination thereof. In some embodiments, the imaging device may include an imaging component, such as an imaging radiation source and a detector. In some embodiments, the imaging radiation source and the therapeutic radiation source may be integrated into one radiation source for imaging and/or treating the subject.

The network 120 may include any suitable network that facilitates the exchange of information and/or data of the medical system 100. In some embodiments, the one or more components (e.g., the medical device 110, the terminal 130, the processing device 140, and the storage device 150) of the medical system 100 may communicate information and/or data with the one or more other components of the medical system 100 via the network 120. For example, the processing device 140 may obtain image data from the medical device 110 via the network 120. As another example, the processing device 140 may obtain a user instruction from the one or more terminals 130 via the network 120. The network 120 may be and/or include a public network (e.g., the Internet), a private network (e.g., a local area network (LAN), and a wide area network (WAN)), a wired network (e.g., a wireless local area network), an Ethernet, a wireless network (e.g., an 802.11 network, and a Wi-Fi network), a cellular network (e.g., a long term evolution (LTE) network), a frame relay network, a virtual private network (VPN), a satellite network, a telephone network, a router, a hub, a switch, a server computer, and/or any combination thereof. Merely by way of example, the network 120 may include a cable network, a wired network, a fiber optic network, a telecommunication network, an intranet, a wireless local area network (WLAN), a metropolitan area network (MAN), a public switched telephone network (PSTN), a Bluetooth network, a Zigbee network, a near field communication (NFC) network, or the like, or any combination thereof. In some embodiments, the network 120 may include one or more network access points. For example, the network 120 may include a wired and/or wireless network access point such as a base station and/or an Internet exchange point, and the one or more components of the medical system 100 may be connected to the network 120 via the wired and/or wireless access point to exchange data and/or information.

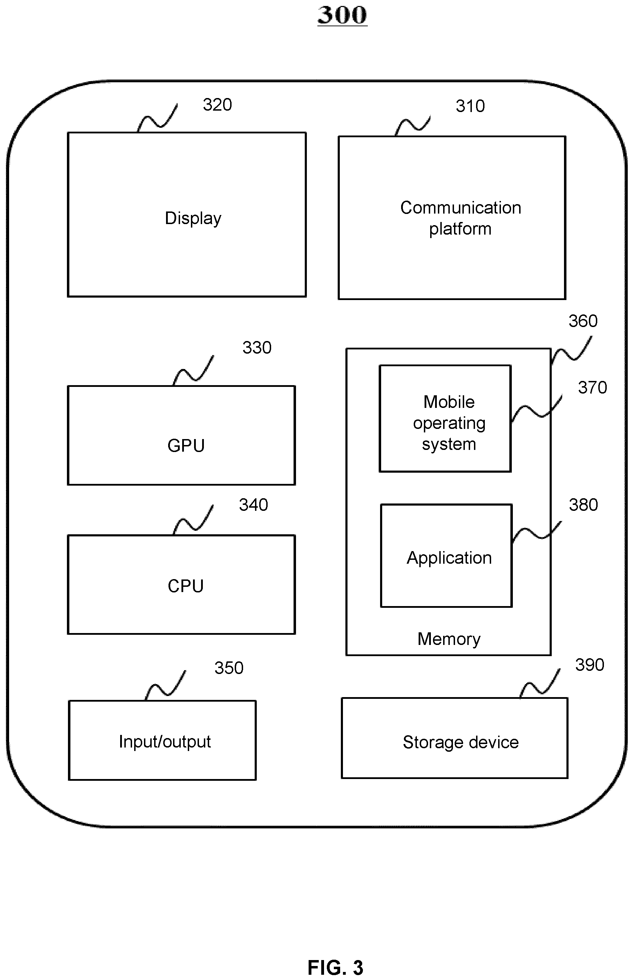

The one or more terminals 130 may include a mobile device 130-1, a tablet computer 130-2, a laptop computer 130-3, or the like, or any combination thereof. In some embodiments, the mobile device 130-1 may include a smart home device, a wearable device, a smart mobile device, a virtual reality (VR) device, an augmented reality (AR) device, or the like, or any combination thereof. Merely by way of example, the one or more terminals 130 may include a mobile device as shown in FIG. 3. In some embodiments, the smart home device may include a smart lighting device, a smart appliance control device, a smart monitoring device, a smart TV, a smart camera, an intercom, or the like, or any combination thereof. In some embodiments, the wearable device may include a bracelet, footwear, glasses, a helmet, a watch, clothing, a backpack, a smart accessory, or the like, or any combination thereof. In some embodiments, the mobile device may include a mobile phone, a personal digital assistant (PDA), a gaming device, a navigation device, a point of sale (POS) device, a laptop computer, a tablet computer, a desktop computer, or the like, or any combination thereof. In some embodiments, the VR device and/or the AR device may include a VR helmet, VR glasses, VR goggles, an AR helmet, AR glasses, AR goggles, or the like, or any combination thereof. In some embodiments, the one or more terminals 130 may be a part of the processing device 140. In some embodiments, the one or more terminals 130 may be a part of the medical device 110.

The processing device 140 may process data and/or information obtained from the medical device 110, the one or more terminals 130, and/or the storage device 150. For example, the processing device 140 may obtain status information of the medical device 110 and a target plan of a target subject. As another example, the processing device 140 may determine a prediction result based on the status information of the medical device and the target plan. As another example, the processing device 140 may determine whether a quality assurance test is passed based on the prediction result. As another example, in response to determining that the quality assurance test is passed, the processing device 140 may control the medical device to perform a target operation on the target subject. In some embodiments, the processing device 140 may be a single server or a server group. The server group may be centralized or distributed. In some embodiments, the processing device 140 may be a local component or a remote component relative to one or more other components of the medical system 100. For example, the processing device 140 may access information and/or data stored in the medical device 110, the one or more terminals 130, and/or the storage device 150 via the network 120. As another example, the processing device 140 may be directly connected with the medical device 110, the one or more terminals 130, and/or the storage device 150 to access the stored information and/or data. In some embodiments, the processing device 140 may be implemented on a cloud platform. Merely by way of example, the cloud platform may include a private cloud, a public cloud, a hybrid cloud, a community cloud, a distributed cloud, an internal cloud, a multi-layer cloud, or the like, or any combination thereof. In some embodiments, the processing device 140 may be implemented by a computing device 200 including one or more components as shown in FIG. 2.

The storage device 150 may be configured to store data, instructions, and/or any other information. In some embodiments, the storage device 150 may store data obtained from the one or more terminals 130 and/or the processing device 140. In some embodiments, the storage device 150 may store data and/or instructions that the processing device 140 may execute or use to execute the exemplary methods described in the specification of this application. In some embodiments, the storage device 150 may include a mass memory, a removable memory, a volatile read-write memory, a read-only memory (ROM), or the like, or any combination thereof. Exemplary mass memories may include a disk, an optical disk, a solid-state drive, etc. Exemplary removable memories may include a flash drive, a floppy disk, an optical disk, a memory card, a compressed disk, a magnetic tape, etc. Exemplary volatile read-write memories may include a random access memory (RAM). Exemplary RAMs may include a dynamic random access memory (DRAM), a double data rate synchronous dynamic random access memory (DDR SDRAM), a static random access memory (SRAM), a thyristor random access memory (T-RAM), a zero capacitance random access memory (Z-RAM), etc. Exemplary ROMs may include a mask ROM (MROM), a programmable ROM (PROM), an erasable programmable ROM (EPROM), an electrically erasable programmable ROM (EEPROM), a compact disk ROM (CD-ROM), a digital versatile disk ROM, etc. In some embodiments, the storage device 150 may be implemented on the cloud platform. Merely by way of example, the cloud platform may include the private cloud, the public cloud, the hybrid cloud, the community cloud, the distributed cloud, the internal cloud, the multi-layer cloud, or the like, or any combination thereof.

In some embodiments, the storage device 150 may be connected to the network 120 to communicate with one or more other components (e.g., the processing device 140, and the one or more terminals 130) of the medical system 100. The one or more components of the medical system 100 may access data or instructions stored in the storage device 150 via the network 120. In some embodiments, the storage device 150 may be directly connected with or communicate with the one or more other components (e.g., the processing device 140, and the one or more terminals 130) of the medical system 100. In some embodiments, the storage device 150 may be a part of the processing device 140.

It should be noted that the above description is provided for illustrative purposes only and is not intended to limit the scope of the present disclosure. For those of ordinary skill in the art, various changes and modifications can be made under the guidance of the present disclosure. The features, structures, methods and other features of the exemplary embodiments described in the present disclosure can be combined in various ways to obtain additional and/or alternative exemplary embodiments. However, these changes and modifications do not deviate from the scope of the present disclosure.

FIG. 2 is a schematic diagram illustrating an exemplary computing device on which a processing device is implemented according to some embodiments of the present disclosure. As shown in FIG. 2, a computing device 200 may include a processor 210, a storage device 220, an input/output 230, and a communication port 240.

The processor 210 may be configured to execute computer instructions (e.g., a program code) and perform functions of the processing device 140 according to the techniques described in the present disclosure. The computer instructions may include, for example, a routine, a program, a subject, a component, a data structure, a process, a module, and a function, which perform specific functions described in the present disclosure. For example, the processor 210 can process data or information obtained from the medical device 110, the storage device 150, the one or more terminals 130, and/or any other component of the medical system 100. In some embodiments, the processor 210 may include one or more hardware processors, such as a microcontroller, a microprocessor, a reduced instruction set computer (RISC), an application-specific integrated circuit (ASIC), an application-specific instruction set processor (ASIP), a central processing unit (CPU), a graphics processing unit (GPU), a physical processing unit (PPU), a microcontroller unit, a digital signal processor (DSP), a field programmable gate array (FPGA), an advanced RISC machine (ARM), a programmable logic device (PLD), any circuit or processor capable of performing one or more functions, or the like, or any combination thereof.

For illustration purposes only, only one processor is described in the computing device 200. However, it should be noted that the computing device 200 disclosed in the present disclosure may also include a plurality of processors. Therefore, the operations and/or process steps performed by one processor disclosed in the present disclosure may also be performed jointly or separately by the plurality of processors. For example, if in the present disclosure, the processor of the computing device 200 performs operations A and B, it should be understood that operations A and B may also be performed jointly or separately by two or more different processors of the computing device 200 (e.g., a first processor may perform operation A, and a second processor may perform operation B, or the first processor and the second processor may perform the operations A and B together).

The storage device 220 may store data/information obtained from the medical device 110, the storage device 150, the one or more terminals 130, and/or any other component of the medical system 100. In some embodiments, the storage device 220 may include a mass memory, a removable memory, a volatile read-write memory, a read-only memory, or the like, or any combination thereof. For example, the mass memory may include a magnetic disk, an optical disk, a solid-state drive, or the like. The removable memory may include a flash drive, a floppy disk, an optical disk, a memory card, a compact disk, a magnetic tape, or the like. The volatile read-write memory may include a random access memory (RAM). The RAM may include a dynamic RAM (DRAM), a double data rate synchronous dynamic RAM (DDR SDRAM), a static RAM (SRAM), a thyristor RAM (T-RAM), and a zero capacitor RAM (Z-RAM), or the like. The ROM may include a mask ROM (MROM), a programmable ROM (PROM), an erasable programmable ROM (EPROM), an electrically erasable programmable ROM (EEPROM), a compact disk ROM (CD-ROM), and a digital versatile disk ROM, or the like. In some embodiments, the storage device 220 may store one or more programs and/or instructions to execute the exemplary methods described in the present disclosure.

The input/output 230 may input and/or output a signal, data, information, etc. In some embodiments, the input/output 230 enables a user to interact with the processing device 140. In some embodiments, the input/output 230 may include an input device and an output device. Exemplary input devices may include a keyboard, a mouse, a touch screen, a microphone, or the like, or a combination thereof. Exemplary output devices may include a display device, a loudspeaker, a printer, a projector, or the like, or a combination thereof. Exemplary display devices may include a liquid crystal display (LCD), an LED-based display, a flat panel display, a curved screen, a TV device, a cathode ray tube (CRT), a touch screen, or the like, or a combination thereof.

The communication port 240 may be connected to a network (e.g., the network 120) to facilitate data communication. The communication port 240 may establish a connection between the processing device 140 and the medical device 110, the storage device 150, and/or the one or more terminals 130. The connection may be a wired connection, a wireless connection, any other communication connection capable of achieving data transmission and/or reception, and/or any combination thereof. The wired connection may include, cable, an optical cable, a telephone line, or the like, or any combination thereof. The wireless connection may include Bluetooth, Wi-Fi, WiMax, wireless LAN Zigbee, a mobile network (e.g., 3G, 4G, and 5G), or the like, or any combination thereof. In some embodiments, the communication port 240 may be and/or include a standardized communication port, such as an RS232, an RS485, etc. In some embodiments, the communication port 240 may be a specially designed communication port. For example, the communication port 240 may be set according to a digital imaging and communications in medicine (DICOM) protocol.

FIG. 3 is a schematic diagram illustrating exemplary hardware and/or software components of an exemplary mobile device on which a terminal is implemented according to some embodiments of the present disclosure. As shown in FIG. 3, a mobile device 300 may include a communication platform 310, a display 320, a GPU 330, a CPU 340, an input/output 350, a memory 360, and a storage device 390. In some embodiments, any other suitable components, including but not limited to a system bus or a controller (not shown in the figure), may also be included in the mobile device 300. In some embodiments, a mobile operating system 370 (e.g., iOS^{™}, Android^{™}, and Windows Phone^{™}) and one or more applications 380 may be loaded from the storage device 390 into the memory 360 for execution by the CPU 340. The one or more applications 380 may include a browser or any other suitable mobile application for receiving and rendering information related to the medical system 100 or other information from the processing device 140. User interaction with the information may be achieved via the input/output 350 and provided to the processing device 140 and/or one or more other components of the medical system 100 via the network 120.

To implement various modules, units, and functions thereof described in the present disclosure, a computer hardware platform may be used as a hardware platform for one or more components described in the present disclosure. A computer with user interface elements may be used as a personal computer (PC) or any other type of workstation or terminal device. If the computer is properly programmed, the computer may also be used as a server.

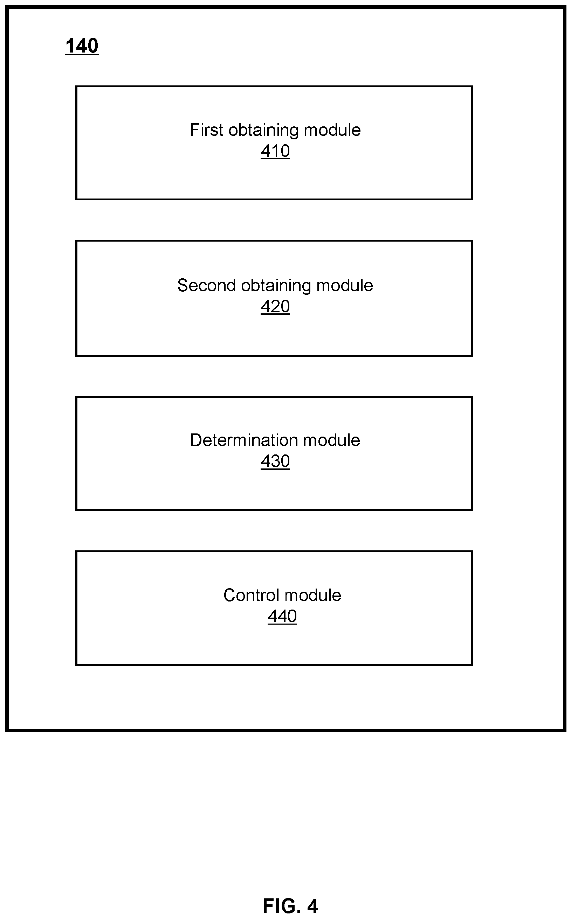

FIG. 4 is a block diagram illustrating an exemplary processing device according to some embodiments of the present disclosure. The processing device 140 may include a first obtaining module 410, a second obtaining module 420, a determination module 430, and a control model 440.

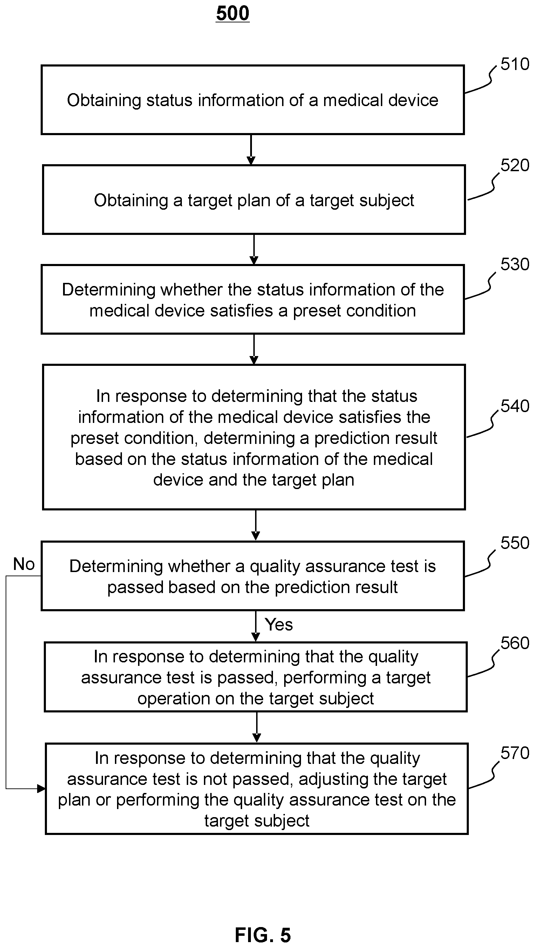

The first obtaining module 410 may be configured to obtain status information of a medical device (e.g., the medical device 110). The status information of the medical device may include at least one of beam information corresponding to a plurality of dose rates, positioning accuracy information of at least one component of the medical device corresponding to a plurality of positions, or an operation error of at least one component of the medical device. More descriptions regarding obtaining the status information of the medical device may be found elsewhere in the present disclosure (e.g., operation 510 in FIG. 5).

The second obtaining module 420 may be configured to obtain a target plan of a target subject. The target plan may include radiotherapy parameters involved in a radiotherapy process. In some embodiments, the second obtaining module 420 may determine a feature related to the complexity of the target plan and/or a target flux map based on the target plan. More descriptions regarding obtaining the status information of the medical device may be found elsewhere in the present disclosure (e.g., operation 520 in FIG. 5).

The determination module 430 may be configured to determine a prediction result based on the status information of the medical device and the target plan. In some embodiments, the determination module 430 may determine whether the status information of the medical device satisfies a preset condition. More descriptions regarding determining whether the status information of the medical device satisfies the preset condition may be found elsewhere in the present disclosure (e.g., operation 530 in FIG. 5). In response to determining that the status information of the medical device satisfies the preset condition, the determination module 430 may determine the prediction result based on the status information of the medical device and the target plan. More descriptions regarding determining the prediction result may be found elsewhere in the present disclosure (e.g., operation 540 in FIG. 5). In some embodiments, the determination module 430 may determine whether a quality assurance test is passed based on the prediction result. More descriptions regarding determining whether the quality assurance test is passed may be found elsewhere in the present disclosure (e.g., operation 550 in FIG. 5). In response to determining that the quality assurance test is not passed, the determination module 430 may adjust the target plan or perform the quality assurance test on the target subject. More descriptions regarding controlling the medical device to implement the target plan on the target subject may be found elsewhere in the present disclosure (e.g., operation 570 in FIG. 5).

The control module 440 may be configured to control one or more components of the medical device. In some embodiments, in response to determining that the quality assurance test is passed, the control module 440 can control the medical device to implement the target plan on the target subject. More descriptions regarding controlling the medical device to implement the target plan on the target subject may be found elsewhere in the present disclosure (e.g., operation 560 in FIG. 5).

It should be noted that the above description of the processing device 140 in the present disclosure is provided for illustrative purposes only and is not intended to limit the scope of the present disclosure. For those of ordinary skill in the art, various changes and modifications can be made according to the description of the present disclosure. However, these changes and modifications do not depart from the scope of the present disclosure. For example, the processing device 140 may also include a storage module (not shown in the figure) for data storage. As another example, the first obtaining module 410 and the second obtaining module 420 may be integrated into one module. As another example, the processing device 140 may further include a training model (not shown in the figure) for training a quality assurance model.

FIG. 5 is a flowchart illustrating an exemplary quality assurance process according to some embodiments of the present disclosure. In some embodiments, at least a portion of a process 500 may be performed by the processing device 140 (e.g., implemented in the computing device 200 shown in FIG. 2). For example, the process 500 may be stored in a storage device (e.g., the storage device 150, the storage device 220, and the storage device 390) in the form of instructions (e.g., application programs) and invoked and/or executed by the processing device 140 (e.g., one or more of the processor 210 shown in FIG. 2, the CPU 340 shown in FIG. 3, or the processing device 140 shown in FIG. 4). The operations of the process shown below are for illustrative purposes only. In some embodiments, the process 500 may be completed using one or more additional operations not described and/or without one or more operations discussed. In addition, the order of the operations of the process 500 shown in FIG. 5 and described below is not intended to be limiting.

In 510, the processing device 140 (e.g., the first obtaining module 410) may obtain status information of a medical device (e.g., the medical device 110).

In some embodiments, the status information of the medical device may reflect a status of the medical device. For example, the status information of the medical device may be expressed by machine operation parameters; the status of the medical device may also be expected by a result of the medical device implementing a radiotherapy plan. In some embodiments, the status information of the medical device may include beam and dose information, positioning accuracy information of at least one component (e.g., a gantry, a scanning table, a radiotherapy radiation source, and a beam limiter) of the medical device, an operation error of at least one component of the medical device, a result of executing a data set using the medical device, or the like, or any combination thereof. In some embodiments, at least one of the beam and dose information, the positioning accuracy information of the at least one component of the medical device, and the operation error of the at least one component of the medical device may be obtained by analyzing the result of executing the data set using the medical device. The beam and dose information may include beam flatness, beam symmetry, beam output correction, beam linearity, a relationship between beam output consistency and a dose rate, or the like, or any combination thereof. The beam output correction refers to monitor unit (MU) correction. For example, after the beam output correction is performed, a central dose at the maximum dose depth under a 10 cm × 10 cm field size may be calibrated to 1MU = 1cGy. In some embodiments, the beam and dose information may include beam information at a plurality of dose rates. For example, the beam and dose information may include the beam flatness, the beam symmetry, the beam linearity, the beam output consistency, etc. at the plurality of dose rates. The plurality of dose rates may be set by a user (e.g., a doctor, and a technician) of the medical system 100 or by the one or more components (e.g., the processing device 140) of the medical system 100 according to different situations.

The positioning accuracy information of the at least one component of the medical device may include information of a rotation isocenter accuracy of the gantry, a rotation isocenter accuracy of the beam limiter, a positioning accuracy of the scanning table, a positioning accuracy of the beam limiter, or the like, or any combination thereof. The positioning accuracy information of the beam limiter may include a positioning accuracy of the leaves of the multi-blade collimator, a movement speed accuracy of the leaves, a positioning repeatability of the leaves, or the like, or any combination thereof. In some embodiments, the positioning accuracy information of at least one component of the medical device may include the positioning accuracy information of the at least one component of the medical device corresponding to a plurality of positions. The plurality of positions may include a plurality of gantry angles, a plurality of beam limiter positions (e.g., a primary collimator position, and a secondary collimator position), a plurality of beam limiter angles (e.g., a primary collimator angle, and a secondary collimator angle), or the like, or any combination thereof. For example, the positioning accuracy information of the beam limiter may include the positioning accuracy of the beam limiter at the plurality of gantry angles. The plurality of positions may be set by a user (e.g., a doctor, and a technician) of the medical system 100 or by one or more components (e.g., the processing device 140) of the medical system 100 according to different situations. For example, the plurality of gantry angles may be set to be 30°, 60°, 120°, 180°, 240°, 300°, etc.

Since the status information of the medical device under different test conditions is usually different (e.g., the beam information under different dose rates and the positioning accuracy information of the beam limiter under different gantry angles are usually different), the status information of the medical device under different test conditions may be fully considered during the quality assurance test, thereby making the result of the quality assurance test more accurate and effective.

The operation error of the one or more components of the medical device may include a systematic error and/or a random error. The systematic error of a component refers to the offset of the component as a whole. For example, the systematic error of the multi-leaf collimator refers to an overall offset of all leaves of the multi-leaf collimator (e.g., all leaves of the multi-leaf collimator are offset by a certain distance in a certain direction as a whole). The random error of a component refers to an offset of a certain subcomponent of the component. For example, the offset of each leaf of the multi-leaf collimator may be different (e.g., the offset of the leaves of the multi-leaf collimator follows a Gaussian distribution). The random error of the multi-leaf collimator refers to an offset of a certain leaf of the multi-leaf collimator (e.g., the leaf is offset by a certain distance in a certain direction).

In some embodiments, the status information of the medical device may include operation errors of the one or more components of the medical device at one or more positions (e.g., the plurality of gantry angles, the plurality of beam limiter positions, and the plurality of beam limiter angles). For example, the status information of the medical device may include the systematic errors and the random errors of the multi-leaf collimator at the plurality of gantry angles, the systematic errors and the random errors of the multi-leaf collimator at the plurality of beam limiter positions and/or angles, or the like, or any combination thereof.

In some embodiments, the processing device 140 may obtain operation information of the medical device. For example, the processing device 140 may obtain historical quality assurance test data of the medical device. Historical quality assurance tests may include quality assurance tests such as daily inspection, weekly inspection, monthly inspection, annual inspection, etc. of the medical device. The historical quality assurance test data may include historical log files and historical EPID images. In some embodiments, the operation information of the medical device may be stored in a storage device (e.g., the storage device 150) or an external storage device of the medical system 100. The processing device 140 may obtain the operation information of the medical device from the storage device (e.g., the storage device 150) or the external storage device of the medical system 100. The processing device 140 may determine the operation error of the at least one component (e.g., the gantry, the radiotherapy radiation source, and the beam limiter) of the medical device based on the operation information of the medical device.

In some embodiments, the processing device 140 may obtain actual operation information and expected operation information of at least one component of the medical device based on the operation information of the medical device. The expected operation information of a component may be operation information (e.g., an expected position, an expected movement speed, an expected movement acceleration, an expected movement distance, and an expected movement direction) of the component set by the user (e.g., the doctor, and the technician) of the medical system 100 through a control device (e.g., the one or more terminals 130) of the medical device. The processing device 140 may determine the operation error of the at least one component of the medical device based on the actual operation information and the expected operation information. For example, the processing device 140 may read an expected operation parameter of the component input by the user and an actual operation parameter of the component from the historical log file of the medical device. As another example, a test device (e.g., a position encoder, a speed sensor, and an acceleration sensor) may be mounted on the component (e.g., the scanning table, and the gantry) of the medical device to detect the actual operation information (e.g., an actual position, an actual movement speed, an actual movement acceleration, an actual movement distance, and an actual movement direction) of the component. Merely by way of example, the user may determine that the expected rotation angle of the gantry is 10° according to a radiotherapy plan, and input the expected rotation angle of the gantry of 10° into the medical device to control the rotation of the gantry. However, due to an operation error of the gantry, the actual rotation angle of the gantry may be 12°. The processing device 140 may determine that the operation error of the gantry is 2° (12°-10°=2°).

In some embodiments, the processing device 140 may determine the operation error of the at least one component of the medical device based on the operation information of the medical device using a first error model. The first error model may be configured to determine the operation error of the at least one component of the medical device according to the operation information of the medical device. For example, the operation information of the medical device may be input into the first error model, and the first error model may output the operation error of at least one component of the medical device. In some embodiments, the processing device 140 may determine the operation error of the at least one component of the medical device based on a simulation image and/or a test image, and a radiotherapy plan of a subject (e.g., a patient) using a second error model. The simulation image may be an EPID image determined based on the radiotherapy plan of the subject using a data simulation algorithm. The test image may be an EPID image collected by an imaging device (e.g., an EPID) based on the radiotherapy plan of the subject. For example, the simulation image and/or the test image, and the radiotherapy plan of the subject may be input into the second error model, and the second error model may output the operation error of the at least one component of the medical device. The first error model and/or the second error model may be a machine learning model. For example, the first error model and/or the second error model may be a convolutional neural network model (CNN), a recurrent neural network model (RNN), a deep neural network model (DNN), a fully convolutional neural network (FCN) model, a generative adversarial network (GAN) model, etc.

As the use time of the medical device increases and the influence of the use environment, the operation status of the medical device usually changes. Different operation statuses of the medical device have a great impact on the result of the quality assurance test. For example, for the same radiotherapy plan, when at least one of the beam and dose information, the positioning accuracy of the component, or the operation error of the component of the medical device changes, the obtained prediction result may be significantly different. Therefore, the current operation status of the medical device during the quality assurance test may be fully considered, so as to make the result of quality assurance test more accurate and effective.

In some embodiments, the processing device 140 may obtain at least one data set. The at least one data set refers to a collection of data related to radiotherapy parameters. In some embodiments, the at least one data set may be determined based on a candidate plan, a limit performance of the at least one component of a medical device, a calculation limit of a dose model, or the like, or any combination thereof. In some embodiments, the at least one data set may include a first data set, a second data set, a third data set, or the like, or any combination thereof. In some embodiments, the at least one data set may include a plurality of radiotherapy plans, and the plurality of radiotherapy plans may include a typical clinical plan. In some embodiments, the at least one data set may also include a radiotherapy plan for examining the limit performance of the at least one component and/or the calculation limit of the dose model.

In some embodiments, the processing device 140 may determine the first data set based on at least one first candidate plan. A value of a first candidate parameter of the at least one first candidate plan may be within a first preset range. The at least one first candidate plan may be a historical radiotherapy plan of a candidate subject. The candidate subject may include a patient subjected to a historical radiotherapy. At least one candidate parameter (e.g., the first candidate parameter, and a second candidate parameter) may include radiotherapy parameters (e.g., the dose rate of the radiation source, a radiation duration, the gantry angle, the gantry movement speed, the gantry movement acceleration, the beam limiter angle, the beam limiter rotation speed, parameters of the leaves of the multi-leaf collimator, a position and/or an angle of a scanning table, a count of radiation fields (or sub-fields), shapes of the radiation fields (or sub-fields), areas of the radiation fields (or sub-fields), perimeters of the radiation fields (or sub-fields), angles of the radiation fields, etc., as described in the operation 520) involved in a radiotherapy process. In some embodiments, the first preset range may be set by the user (e.g., the doctor, and the technician) based on experience. In some embodiments, the first preset range may be determined based on the values of the radiotherapy parameters of a plurality of historical radiotherapy plans. For example, taking the radiotherapy parameter being the count of radiation fields as an example, if the count of historical radiotherapy plans having radiation fields within a range of 1-5 is greater than a preset threshold (e.g., 90%, 95%, and 99%), the first preset range may be within a range of 1-5. In other words, the first data set may be a set of data determined based on the most common radiotherapy plans in a clinical practice, i.e., the values of the radiotherapy parameters in the first data set may be within the range of the values of the corresponding radiotherapy parameters in most historical radiotherapy plans.

In some embodiments, the processing device 140 may directly use the values of the radiotherapy parameters of the at least one first candidate plan as the values of the radiotherapy parameters in the first data set. In some embodiments, the processing device 140 may adjust the values of the radiotherapy parameters of the at least one first candidate plan, and use the adjusted values of the radiotherapy parameters as the values of the radiotherapy parameters in the first data set. In some embodiments, the processing device 140 may input a plurality of first candidate plans into a parameter extraction model. The parameter extraction model may extract the values of the radiotherapy parameters in the plurality of first candidate plans. The processing device 140 may use the extracted values of the radiotherapy parameters as the values of the radiotherapy parameters in the first data set. In some embodiments, the processing device 140 may input the plurality of first candidate plans into the parameter extraction model, and the parameter extraction model may output the first data set. The parameter extraction model refers to a machine learning model or an algorithm for extracting the values of the radiotherapy parameters in the at least one candidate plan or determining the at least one data set (e.g., the first data set and the second data set) according to the at least one candidate plan (e.g., the first candidate plan and the second candidate plan).

In some embodiments, the processing device 140 may determine the second data set based on at least one second candidate plan. A value of a second candidate parameter of the at least one second candidate plan may exceed a second preset range. The at least one second candidate plan may be a historical radiotherapy plan of the candidate subject. The first preset range and the second preset range may be the same or different. In some embodiments, the second preset range may be set by the user (e.g., the doctor, and the technician) based on experience. In some embodiments, the second preset range may also be determined based on the values of the radiotherapy parameters of a plurality of historical radiotherapy plans. For example, taking the radiotherapy parameter being the count of radiation fields as an example, if the count of historical radiotherapy plans having radiation fields within a range of 1-5 is greater than the preset threshold (e.g., 90%, 95%, and 99%), the second preset range may be greater than 5. As another example, the second preset range may be determined based on the values of the radiotherapy parameters of some special types of radiotherapy plans (e.g., a large field radiotherapy plan). As another example, if the value of a certain radiotherapy parameter of a historical radiotherapy plan based on a certain radiotherapy purpose needs to be relatively high or low (e.g., a relatively small sub-field area, and a relatively high degree of offset of the sub-field from a beam center), the value of the radiotherapy parameter of the historical radiotherapy plan may be used as the value of the radiotherapy parameter in the second data set. That is to say, the second data set may be a set of data determined based on radiotherapy plans that are not common in the clinical practice, i.e., the values of the radiotherapy parameters in the second data set may exceed the range of the values of the corresponding radiotherapy parameters of most historical radiotherapy plans. The second data set may be determined similarly to the first data set, which is not repeated here. For example, the processing device 140 may directly use the values of the radiotherapy parameters of the at least one second candidate plan as the values of the radiotherapy parameters in the second data set. As another example, the processing device 140 may determine the values of the radiotherapy parameters in the at least one second data set based on a plurality of second candidate plans using the parameter extraction model.

In some embodiments, the processing device 140 may determine the third data set based on the limit performance of the at least one component of the medical device and/or parameter information (e.g., the calculation limit of the dose model) of the dose model. For example, the third data set may include limit performance parameters of the at least one component of the medical device and/or parameters related to the calculation limit of the dose model. The limit performance of the medical device may include a maximum movement speed, a maximum movement acceleration, a limit movement position, or the like, or any combination thereof of the at least one component (e.g., the radiation source, the gantry, the scanning table, and the leaves of the multi-leaf collimator) of the medical device. Since the performance of each of the at least one component of the medical device cannot exceed the limit performance, values of the parameters related to the radiotherapy process in the third data set need to be reasonably set according to the limit performance of the at least one component of the medical device.

The dose model may include an algorithm or a model that can simulate and calculate a dose-related result (e.g., an EPID image, and a dose distribution) based on the values of the radiotherapy parameters. In some embodiments, the parameter information of the dose model may include a type of the dose model (e.g., a type of a dose algorithm), a parameter of the dose model (e.g., a parameter of the dose algorithm), or the like, or any combination thereof. The dose model (or the dose algorithm) may include a Monte Carlo dose model, a pencil beam algorithm, a convolution algorithm, a machine learning algorithm, or the like, or any combination thereof.

In some embodiments, the parameter information of the dose model may include parameters of models of one or more components of the medical device. For example, the parameter information of the dose model may include parameters of an accelerator model (e.g., a beam model). The accelerator model may be configured to determine an energy (e.g., an absorbed dose) deposited by the beam in a target volume and an organ at risk. In some embodiments, the parameters of the accelerator model may include relevant parameters (e.g., position parameters) of components such as a primary collimator, a homogenizer, a secondary collimator, etc. For example, the parameter information of the dose model may include an offset of the multi-leaf collimator, transmittance of the multi-leaf collimator, a size of a leaf -tip structure of the leaves of the multi-leaf collimator, a width and transmittance of a tongue-and-groove structure of the leaves of the multi-leaf collimator, or the like, or any combination thereof.

The dose model can simplify the process of dose calculation and improve the efficiency of dose calculation. However, the use of different types of dose models or different dose model parameters may have a great impact on the result of the quality assurance test. For example, for the same radiotherapy plan, when different dose model parameters are used, the predict results may be significantly different. Therefore, the parameter information of the dose model during the quality assurance test may be fully considered, so as to make the result of the quality assurance test more accurate and effective.

In some embodiments, the calculation limit of the dose model may reflect the calculation capability of the dose model. If the calculation capability of the dose model is poor (e.g., the calculation accuracy is low), a pass rate of the dose model in the test of the calculation limit may be low, so the test of the calculation limit may reflect the calculation capability of the dose model. For example, the calculation limit of the dose model may include the calculation accuracy of the dose model for a parameter value that is prone to calculation errors. For example, since the rays in the radiation field are usually non-uniformly distributed, the dose rate of the rays near a boundary of the radiation field is low, and the non-uniform distribution of the rays may lead to an error in the dose calculation (especially the dose calculation at the boundary of the radiation field). Therefore, the accuracy of the dose model for the dose calculation at the boundary of the radiation field may reflect the calculation capability of the dose model.

In some embodiments, the at least one data set may also include other types of data sets. For example, a user (e.g., the doctor, and the technician) of a medical system 100 may set the values of the radiotherapy parameters in the at least one data set based on experience. As another example, the user (e.g., the doctor, and the technician) of the medical system 100 may set a range of the value of each radiotherapy parameter in the data set. The processing device 140 may randomly set the value of the radiotherapy parameter based on the range of the value of the radiotherapy parameter to generate the at least one data set. As another example, the processing device 140 may randomly select a historical radiotherapy plan from a database, and use the values of the radiotherapy parameters of the selected historical radiotherapy plan as the values of the radiotherapy parameters in the data set.

In some embodiments, the processing device 140 may determine an actual test result based on the at least one data set. For example, the values of the radiotherapy parameters in the first data set may be implemented when a gantry angle is within a range of 0°-30°, the values of the radiotherapy parameters in the second data set may be implemented when the gantry angle is within a range of 30°-60°, and the values of the radiotherapy parameters in the third data set may be implemented when the gantry angle is within a range of 60°-120°. In some embodiments, the actual test result may be determined based on the at least one data set using the medical device and/or a test device. The actual test result may include an image, a dose distribution, or the like, or any combination thereof. The dose distribution refers to a spatial distribution of energy deposition of radioactive particles such as photons or charged particles irradiated into a subject. In some embodiments, the dose distribution may include a dose delivered to one or more parts of the subject and/or an absorbed dose absorbed by one or more parts of the subject. In some embodiments, the test device may include an imaging device (e.g., the EPID). The actual test result may include a test image collected by the imaging device (e.g., the EPID) based on the at least one data set. For example, the radiotherapy process may be implemented according to the parameters of the radiotherapy plan in the at least one data set, and the EPID may receive the irradiation of the radiation field to generate a portal image. In this case, the test image may be the portal image. In some embodiments, the processing device 140 may reconstruct the portal image into a dose distribution image. In some embodiments, the test device may include a third-party device (e.g., a phantom, and a dose measurement device). The actual test result may include a measured dose distribution detected by the dose measurement device based on the at least one data set. For example, the phantom may be placed on the scanning table, the radiotherapy process may be implemented on the phantom according to the values of the radiotherapy parameters in the at least one data set, and the measured dose distribution in the phantom may be detected by the dose measurement device. As another example, the radiotherapy process may be implemented on the phantom according to the parameters of the radiotherapy plan in at least one data set, and a test image (e.g., the portal image or the dose distribution image converted from the portal image) of the phantom may be obtained by the EPID.

In some embodiments, the processing device 140 may determine a predicted test result based on the at least one data set. The predicted test result may include an image, a dose distribution, or the like, or any combination thereof. In some embodiments, the processing device 140 may obtain simulation scan data based on the at least one data set using the dose model or other data simulation algorithm. The processing device 140 may generate the simulation image based on the simulation scan data. In some embodiments, the processing device 140 may obtain a simulated dose distribution based on the at least one data set using the dose model. In some embodiments, the predicted test result may also include a planned dose distribution. The planned dose distribution refers to an expected dose distribution. The planned dose distribution may be set by the user (e.g., the doctor, and the technician) of the medical system 100 or by the at least one component (e.g., the processing device 140) of the medical device 110 according to an actual situation.

Further, the processing device 140 may determine a data set execution result of the medical device based on the actual test result and the predicted test result. The data set execution result may include a difference between the actual test result and the predicted test result. For example, the processing device 140 may determine a difference between the measured dose distribution and the simulated dose distribution (or the planned dose distribution). In some embodiments, the difference between the actual test result and the predicted test result obtained by executing the at least one data set using the medical device may reflect the status information of the medical device. For example, the smaller the difference between the actual test result and the predicted test result obtained by executing the at least one data set using the medical device, the better the status of the medical device, and the higher the accuracy of executing the radiotherapy plan using the medical device. In addition, the difference between the actual test result and the predicted test result obtained by executing different types of data sets using the medical device may reflect different types of status information of the medical device. For example, by executing the first data set, the accuracy of the medical device executing the common radiotherapy plans in the clinical practice may be reflected, and by executing the second data set and/or the third data set, the accuracy of the medical device executing the uncommon radiotherapy plans in the clinical practice may be reflected. In some embodiments, a plurality of execution results (e.g., a plurality of differences between the actual test results and the predicted test results) may be obtained by executing the same data set (e.g., the second data set, and the third data set) multiple times using the medical device. The differences between the plurality of execution results may reflect changes in the status information of the medical device. For example, during the factory inspection of the medical device, a first execution result may be determined by executing the third data set using the medical device. After the medical device is used for a period of time, a second execution result may be determined by executing the third data set using the medical device again. A difference between the first execution result and the second execution result may reflect a change between the current status of the medical device and the status of the medical device during the factory inspection.

In some embodiments, the first data set and/or the second data set may include a plurality of data subsets. The plurality of data subsets may be configured to correspond to different types of radiotherapy plans. Different types of radiotherapy plans may be categorized based on a part receiving radiotherapy, the type of target volume (e.g., a tumor), and/or a type of radiotherapy technique (e.g., a two-dimensional conformal radiotherapy, a three-dimensional conformal radiotherapy, an intensity modulated radiation therapy, an image-guided radiotherapy, a bio-guided radiotherapy, a dose-guided radiotherapy, an online adaptive radiotherapy, etc.).

In 520, the processing device 140 (e.g., the second obtaining module 420) may obtain a target plan of a target subject.

The target subject refers to a patient to be treated with radiotherapy. The target plan (e.g., a target radiotherapy plan) indicates how to perform the radiotherapy plan on the target subject. For example, the target plan may include radiotherapy parameters involved in a radiotherapy process. For example, the radiotherapy parameters may include a dose rate (i.e., MUs/min) of a radiation source, a radiation duration, a gantry angle, a gantry movement speed, a gantry movement acceleration, a beam limiter angle, a beam limiter rotation speed, parameters (e.g., a position, a movement speed, a movement acceleration, and a movement direction) of leaves of a multi-leaf collimator, a position and/or an angle of a scanning table, a count of radiation fields (or sub-fields), shapes of the radiation fields (or sub-fields), areas of the radiation fields (or sub-fields), perimeters of the radiation fields (or sub-fields), angles of the radiation fields, or the like, or any combination thereof. In some embodiments, the target plan may include an intensity modulated radiotherapy (IMRT) plan, a dynamic rotation intensity modulated radiotherapy (uARC) plan, an image-guided radiotherapy plan, a bio-guided radiotherapy plan, a dose-guided radiotherapy plan, an online adaptive radiotherapy plan, or the like, or any combination thereof.

In some embodiments, the sub-fields are small radiation fields with a certain shape through which rays can pass, and the shapes of a plurality of sub-fields may be superposed (which may be referred to as a radiation field) to match the shape of the target volume. The count of the sub-fields may be determined according to a type of radiotherapy device, and a position, an area, a shape, etc. of the target volume. The shapes of the sub-fields may be achieved by the positions of the leaves of the multi-leaf collimator and/ or a tungsten gate. During radiotherapy, the sub-fields of different shapes and areas may be formed by a change in a leaf spacing, a movement direction, a movement speed, etc. of the multi-leaf collimator. The dose rate of the radiation source refers to an intensity of a particle beam or beam generated by the radiation source passing through the sub-fields to irradiate the target volume per unit time. The radiation duration refers to a duration of the particle beam or the beam generated by the radiotherapy device (e.g., a therapeutic radiation source) passing through the sub-fields to irradiate the target volume. In some embodiments, the dose distribution may be determined based on the dose rate and the radiation duration at a specific angle. The angle of the radiation field refers to an incident angle of the particle beam or the beam generated by the radiotherapy device (e.g., the therapeutic radiation source) to irradiate the target volume. The gantry angle may be related to an orientation of a radiation source (e.g., a therapeutic radiation source) relative to an isocenter of the radiotherapy device. For example, the gantry angle may be an angle between a vertical direction and a beam axis direction of a radiation beam emitted from the radiation source. For example, the therapeutic radiation source may be detachably or fixedly connected to the gantry, and when the gantry rotates around a rotation axis of the gantry, the therapeutic radiation source may rotate with the gantry, such that the subject on the scanning table may be imaged and/or treated from different gantry angles.

In some embodiments, the processing device 140 may obtain a planning image of the target subject. The planning image refers to an image used to formulate the target plan of the target subject. The planning image may include information related to a treatment region of the target subject. For example, the planning image may show a tumor of the target subject and tissues and/or organs near the tumor. The planning image may be a CT image, an MRI image, etc. In some embodiments, the planning image may be a historical medical image of the target subject. In some embodiments, the planning image may be the latest medical image obtained before a radiotherapy operation is performed on the target subject. The user (e.g., the doctor) of the processing device 140 or the medical system 100 may determine the target plan based on the planning image of the target subject.

In some embodiments, the target plan of the target subject may be stored in a storage device (e.g., the storage device 150) or an external storage device of the medical system 100, and the processing device 140 may obtain the target plan from the storage device (e.g., the storage device 150) or the external storage device of the medical system 100.

In some embodiments, the processing device 140 may determine a feature related to the complexity of the target plan based on the target plan of the target subject. The complexity of the target plan may reflect the difficulty of performing radiotherapy operations on the target subject based on the radiotherapy parameters involved in the target plan. In some embodiments, the complexity of the target plan may be related to the values of the radiotherapy parameters in the radiotherapy process based on the target plan. For example, the complexity of the target plan may relate to the values of the radiotherapy parameters, a change of the values of the radiotherapy parameters, a change rate of the values of the radiotherapy parameters, or the like, or any combination thereof.

In some embodiments, the larger the values of the radiotherapy parameters of the target plan, the higher the complexity of the target plan. For example, the more the sub-fields during the radiotherapy process, the higher the complexity of the target plan. In some embodiments, the greater the change of the values of the radiotherapy parameters of the target plan, the higher the complexity of the target plan. For example, the greater the change of the dose rate during radiotherapy, the higher the complexity of the target plan. In some embodiments, the higher the change rate of the values of the radiotherapy parameters of the target plan, the higher the complexity of the target plan. For example, the higher the change rate of the gantry angle during radiotherapy, the higher the complexity of the target plan.

In some embodiments, the feature related to the complexity of the target plan may include an irregularity of the radiation beam of the radiotherapy device, a product of an MU corresponding to a radiation field and an area of the radiation field area, a perimeter of the radiation field, a proportion of small fields, a degree of deviation of the radiation field from an isocenter point of the medical device, a change in the positions of the collimator leaves between adjacent sub-fields, or the like, or any combination thereof. The proportion of the small fields refers to a ratio of a count of collimator leaves whose radiation fields are less than a threshold (e.g., 1 cm, and 2 cm) during field opening to a total count of collimator leaves participating in the field opening. For example, assuming that there are 10 pairs of collimator leaves participating in the field opening, of which the radiation fields of 2 pairs of collimator leaves are less than the threshold (e.g., 2 cm), the proportion of the small fields is one-fifth. In some embodiments, the higher the irregularity of the radiation beam of the radiotherapy device, the greater the product of the count of MU corresponding to the radiation field and the area of the radiation field, the smaller the perimeter of the radiation field, the higher the proportion of the small fields, the higher the degree of deviation of the radiation field from the isocenter point of the medical device, and/or the greater the change in the positions of the collimator leaves between the adjacent sub-fields, the higher the complexity of the target plan.

In some embodiments, the processing device 140 may determine a target flux map based on the target plan of the target subject. In some embodiments, the target flux map may be configured to characterize a flux distribution of a beam flow through the beam limiter. For example, the target flux map may represent an expected radiation intensity distribution of the beam planned to be delivered to the target volume of the subject in radiotherapy. In some embodiments, for each radiation beam, the radiotherapy device may perform radiotherapy on the target subject on the scanning table from a plurality of gantry angles based on the target plan of the target subject. For each of the plurality of gantry angles, the processing device 140 may determine a plurality of first sub-flux maps corresponding to the plurality of sub-fields based on unit time radiation intensities and radiation time corresponding to the plurality of sub-fields under the gantry angle. The processing device 140 may determine a plurality of second sub-flux maps corresponding to the plurality of gantry angles based on the plurality of first sub-flux maps. For example, the processing device 140 may determine the plurality of second sub-flux maps by weighted fusion of the plurality of first sub-flux maps. Further, the processing device 140 may determine the target flux map corresponding to the radiation beam based on the plurality of second sub-flux maps corresponding to the plurality of gantry angles. For example, the processing device 140 may determine the target flux map corresponding to the radiation beam by weighted fusion of the plurality of second sub-flux maps. In some embodiments, since the target flux map may be determined based on the radiotherapy parameters using the dose model, the target flux map may also reflect information (e.g., parameters related to the radiation fields or sub-fields) related to the radiotherapy parameters and the parameter information of the dose model.

In 530, the processing device 140 (e.g., the determination module 430) may determine whether the status information of the medical device satisfies a preset condition.

In some embodiments, the processing device 140 may determine whether beam information (e.g., beam flatness, beam symmetry, beam linearity, beam output consistency, etc.) at one or more dose rates satisfies a corresponding preset condition (e.g., a flatness threshold, a symmetry threshold, a linearity threshold, and an output consistency threshold.). If the beam information (e.g., the beam flatness, the beam symmetry, the beam linearity, the beam output consistency, etc.) at the one or more dose rates satisfies the corresponding preset condition (e.g., greater than the flatness threshold, greater than the symmetry threshold, greater than the linearity threshold, and greater than the output consistency threshold), the processing device 140 may determine that the beam information satisfies the preset condition and the check of the status information of the medical device is passed.

In some embodiments, the processing device 140 may determine whether positioning accuracy information of at least one component of the medical device is greater than a corresponding accuracy threshold. If the positioning accuracy information of the at least one component is greater than the corresponding accuracy threshold, the processing device 140 may determine that the positioning accuracy information of the at least one component satisfies the preset condition, and the check of the status information check of the medical device is passed.

In some embodiments, the processing device 140 may determine whether an operation error of the at least one component of the medical device is less than a corresponding error threshold. If the operation error of the at least one component is less than the corresponding error threshold, the processing device 140 may determine that the operation error of the at least one component satisfies the preset condition, and the check of the status information of the medical device is passed.

In some embodiments, the processing device 140 may determine whether the status information of the medical device satisfies the preset condition based on a data set execution result (e.g., a difference between an actual test result and a predicted test result) of the medical device. For example, the processing device 140 may determine whether the data set execution result is passed based on a difference between a test image collected by an imaging device (e.g., an EPID) and a simulation image and a first difference threshold, thereby determining whether the status information of the medical device satisfies the preset condition. In some embodiments, a difference between a first image (e.g., the test image) and a second image (e.g., the simulation image) may be represented by a difference between an average grayscale value of pixels or voxels of the first image and an average grayscale value of pixels or voxels of the second image. If the difference between the test image and the simulation image is greater than the first difference threshold, the processing device 140 may determine that the data set execution result is not passed, and determine that the status information of the medical device does not satisfy the preset condition. If the difference between the test image and the simulation image is less than the first difference threshold, the processing device 140 may determine that the data set execution result is passed, and determine that the status information of the medical device satisfies the preset condition. As another example, the processing device 140 may determine whether the data set execution result is passed based on a difference between a measured dose distribution and a planned dose distribution, thereby determining whether the status information of the medical device satisfies the preset condition. In some embodiments, the processing device 140 may determine whether the status information of the medical device satisfies the preset condition based on a pass rate of the data set execution results of the medical device. If the pass rate of the data set execution results of the medical device is greater than a pass rate threshold, the processing device 140 may determine that the status information of the medical device satisfies the preset condition.

In some embodiments, the same data set (e.g., a second data set, and a third data set) may be executed multiple times using the medical device to obtain a plurality of execution results (e.g., multiple differences between the actual test results and the predicted test results). The processing device 140 may determine whether the status information of the medical device satisfies the preset condition based on a plurality of test results. For example, during the factory inspection of the medical device, a first execution result (e.g., an EPID image, and a dose distribution) may be determined by executing the third data set using the medical device. After the medical device is used for a period of time, a second execution result (e.g., the EPID image, and the dose distribution) may be determined by executing the third data set using the medical device again. The processing device 140 may determine whether a difference between the first execution result and the second execution result is less than a second difference threshold. If the difference between the first execution result and the second execution result is less than the second difference threshold, the processing device 140 may determine that the status information of the medical device satisfies the preset condition. In some embodiments, for the second data set and the third data set, the processing device 140 may determine whether the status information of the medical device satisfies the preset condition based on a change (e.g., a difference between a current pass rate of the data set execution result and a historical pass rate of the data set execution result) in the pass rate of the data set execution result of the medical device. If the change in the pass rate of the data set execution result of the medical device is less than a pass rate change threshold, the processing device 140 may determine that the status information of the medical device satisfies the preset condition.

In some embodiments, the preset condition (e.g., the flatness threshold, the symmetry threshold, the linearity threshold, the output consistency threshold, the accuracy threshold, the error threshold, the first difference threshold, the second difference threshold, the pass rate threshold, and the pass rate change threshold) may be a system recommended value or a system default value. In some embodiments, the preset condition may be manually set by a user (e.g., the doctor, and the technician) of the medical system 100 or automatically set by one or more components (e.g., the processing device 140) of the medical system 100 according to different situations.

In some embodiments, since the difficulty of changing different status information of the medical device is different, the checking frequency of different status information is also different. For some parameters of the medical device that are easy to change (e.g., an accelerator output, and a positioning accuracy of a multi-leaf grating), the checking frequency needs to be high (e.g., once a day, and once a week) to ensure the accuracy of the radiotherapy process. For some parameters of the medical device that are not easy to change (e.g., a positioning accuracy of a gantry rotation, an isocenter accuracy, and a positioning accuracy of a scanning table), the checking frequency may be relatively low (e.g., once a month, and once a year) to save the total time for a quality assurance test, thereby improving the overall efficiency of the quality assurance test.

In some embodiments, since the accuracy of the medical device in executing the common radiotherapy plan in the clinical practice can be reflected by executing the first data set, the checking frequency of the status information of the medical device based on the first data set may be relatively high (e.g., once a day, and once a week). Since the accuracy of the medical device in executing the radiotherapy plan that is not common in the clinical practice can be reflected by executing the second data set and/or the third data set, the checking frequency of the status information of the medical device based on the second data set and/or the third data set may be relatively low (e.g., once a month, and once a year). In some embodiments, the user may set the checking frequency of each kind of status information of the medical device based on experience and actual needs.

In 540, in response to determining that the status information of the medical device satisfies the preset condition, the processing device 140 (e.g., the determination module 430) may determine a prediction result based on the status information of the medical device and the target plan.

In some embodiments, the processing device 140 may determine the prediction result based on the status information of the medical device and the target plan using a quality assurance model. The quality assurance model refers to a machine learning model or an algorithm used to determine the prediction result based on the status information of the medical device and the target plan of the target subject. The prediction result may include a predicted EPID image, a gamma pass rate, a dose distribution (e.g., a dose volume histogram), a reason why the quality assurance test is not passed, a target adjustment mode, or the like, or any combination thereof. In some embodiments, the prediction result may also include a dose measurement result of a dose measurement device. The dose measurement device may include a dose film, a water tank, a rotating irradiation dosimeter (Arccheck), a multi-sequence plane dosimeter (MapCheck), etc.

In some embodiments, the processing device 140 may obtain a medical image (e.g., scan data) of the target subject, and use the medical image of the target subject as one of inputs of the quality assurance model such that the quality assurance model may output a three-dimensional dose distribution in the target subject. For example, the processing device 140 may input the status information of the medical device, the target plan of the target subject, and the medical image of the target subject into the dose assurance model, and the dose assurance model may output the three-dimensional dose distribution (e.g., the dose volume histogram) in the target subject. The medical image of the target subject may include a CT image, a PET image, an MRI image, or the like, of the target subject, or any combination thereof. In some embodiments, the medical image of the target subject may be a medical image obtained during historical imaging or treatment of the target subject. In some embodiments, the medical image of the target subject may be the latest medical image obtained before a current radiotherapy operation is performed on the target subject (e.g., a few days ago, and a few hours ago), which may reflect a current status of the target subject. For example, the medical image of the target subject may be a planning image used to determine the target plan. As another example, the medical image of the target subject may be a positioning image of the target subject before each fractionated treatment. According to some embodiments of the present disclosure, using the medical image of the target subject as one of the inputs of the quality assurance model can improve the accuracy and rationality of the prediction result (e.g., the dose distribution) output by the quality assurance model, while making the prediction result more clinically meaningful.

In some embodiments, the quality assurance model may include a first model and a second model. The first model may be configured to determine the dose distribution (e.g., a two-dimensional dose distribution, and a three-dimensional dose distribution), the predicted EPID image, and/or the gamma pass rate according to the status information of the medical device, the target plan of the target subject, the parameter information of the dose model, the medical image of the target subject, or the like, or any combination thereof. In some embodiments, when an output of the first model is the predicted EPID image, the target flux map of the target subject needs to be one of the inputs of the first model. For example, the status information of the medical device and the target flux map may be input into the first model, and the first model may output the predicted EPID image. As another example, the status information of the medical device, the target flux map, and the feature related to the complexity of the target plan may be input into the first model, and the first model may output the predicted EPID image. As another example, the status information of the medical device, the target flux map, and the parameter information of the dose model may be input into the first model, and the first model may output the predicted EPID image. As another example, the status information of the medical device, the target flux map, the features related to the complexity of the target plan, and the parameter information of the dose model may be input into the first model, and the first model may output the predicted EPID image. As another example, at least one of the target flux map and the feature related to the complexity of the target plan, and the status information of the medical device may be input into the first model, and the first model may output the gamma pass rate. As another example, at least one of the target flux map or the feature related to the complexity of the target plan, the status information of the medical device, and the parameter information of the dose model may be input into the first model, and the first model may output the gamma pass rate. As another example, the status information of the medical device, the target plan of the target subject, and the medical image of the target subject may be input into the first model, and the first model may output the dose distribution. In some embodiments, when the target flux map is used as the input of the quality assurance model, since the target flux map can reflect the information (e.g., parameters related to the radiation fields or sub-fields) related to the radiotherapy parameters and the parameter information of the dose model, the information related to the radiotherapy parameters and the parameter information of the dose model may not be used as the input of the quality assurance model.

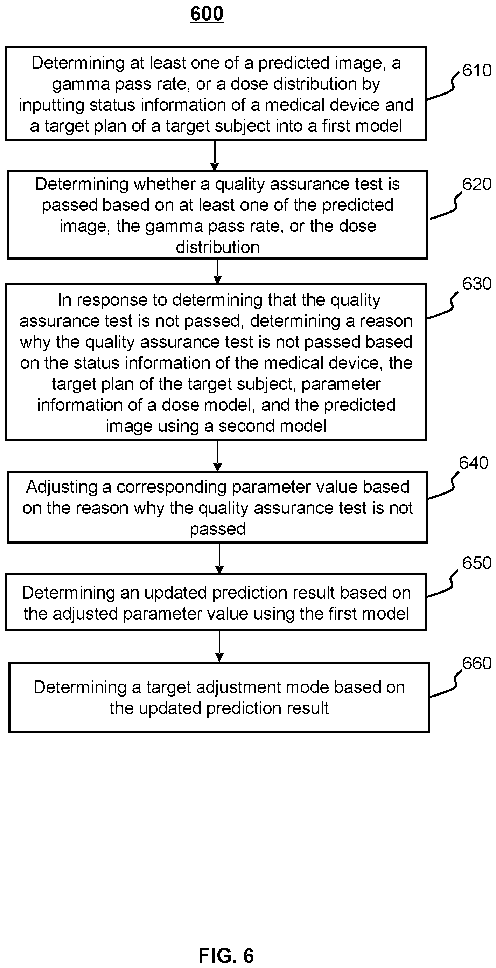

The second model may be configured to determine the reason why the quality assurance test is not passed and/or a target adjustment mode based on the status information of the medical device, the target plan of the target subject, the parameter information of the dose model, the predicted E PID image, the medical image of the target subject, or the like, or any combination thereof. For example, the predicted EPID image (and/or a calculated EPID image), the status information of the medical device, and the target plan (e.g., the target flux map and/or the feature related to the complexity of the target plan) of the target subject may be input into the second model, and the second model may output the reason why the quality assurance test is not passed. As another example, the predicted EPID image (and/or the calculated EPID image), the status information of the medical device, the target plan of the target subject (e.g., the target flux map and/or the feature related to the complexity of the target plan), and the parameter information of the dose model may be input into the second model, and the second model may output the reason why the quality assurance test is not passed. More descriptions regarding determining the prediction result may be found elsewhere in the present disclosure (e.g., FIG. 6 and related descriptions thereof).

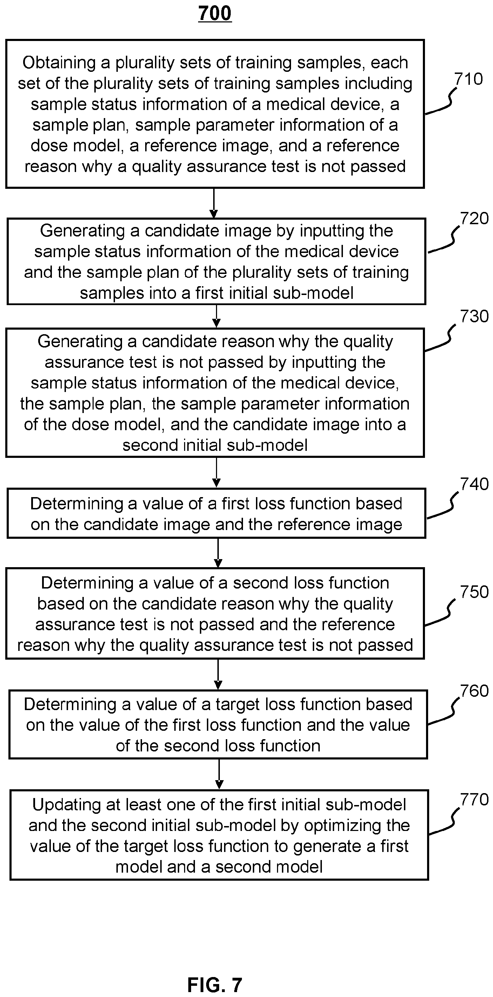

In some embodiments, the quality assurance model may be a machine learning model. For example, the quality assurance model may be a convolutional neural network model (CNN), a recurrent neural network model (RNN), a deep neural network model (DNN), a fully convolutional neural network (FCN) model, a generative adversarial network (GAN) model, etc. In some embodiments, the quality assurance model may be generated by training an initial model based on a plurality sets of training samples using a training algorithm. Exemplary training algorithms may include a gradient descent algorithm, a Newton algorithm, a quasi-Newton algorithm, a conjugate gradient algorithm, a generative adversarial learning algorithm, etc. More descriptions regarding the training process of the quality assurance model may be found elsewhere in the present disclosure (e.g., FIG. 7 and related descriptions thereof).

In some embodiments, the processing device 140 may determine the prediction result based on the status information of the medical device and the target plan using an analytical algorithm. For example, one or more of the status information of the medical device, the target plan, the medical image of the target subject, and the parameter information of the dose model may be used as independent variables in a dose calculation empirical equation, and the prediction result may be used as a dependent variable in the dose calculation empirical equation. The dose calculation empirical equation may be determined based on historical radiotherapy data and/or simulation experiment data.

In 550, the processing device 140 (e.g., the determination module 430) may determine whether a quality assurance test is passed based on the prediction result.

For example, the processing device 140 may determine whether the quality assurance test is passed based on the gamma pass rate and a first threshold. If the gamma pass rate is less than the first threshold, the processing device 140 may determine that the quality assurance test is not passed. If the gamma pass rate is greater than or equal to the first threshold, the processing device 140 may determine that the quality assurance test is passed. As another example, the processing device 140 may determine whether the quality assurance test is passed based on the difference between the predicted image and the calculated image and a second threshold. If the difference between the predicted image and the calculated image is greater than the second threshold, the processing device 140 may determine that the quality assurance test is not passed. If the difference between the predicted image and the calculated image is less than or equal to the second threshold, the processing device 140 may determine that the quality assurance test is passed.

In some embodiments, the target subject may include a plurality of regions of interest (ROI), and the prediction result may include dose distributions (e.g., the dose volume histogram (DVH)) corresponding to the plurality of ROIs. The plurality of ROIs of the target subject may include a planning target volume (PTV), an organ at risk (OAR), other organs or tissues, or the like, of the target subject, or any combination thereof. The processing device 140 may obtain a weight value corresponding to each of the plurality of ROIs. The weight value corresponding to each of the plurality of ROIs may reflect the importance of a dose difference (or the gamma pass rate) between the planned dose distribution and the predicted dose distribution of the ROI in determining whether the quality assurance test is passed. For example, the higher the weight value corresponding to the ROI, the higher the importance of the dose difference (or the gamma pass rate) between the planned dose distribution and the predicted dose distribution of the ROI in determining whether the quality assurance test is passed. In some embodiments, the weight values corresponding to the OAR and the PTV are usually high to ensure that a tumor lesion region (i.e., the PTV) can receive the expected dose of radiation as much as possible during radiotherapy, which can also reduce the damage to the surrounding normal tissues (i.e., the OAR) as much as possible.

The processing device 140 may determine whether the quality assurance test is passed based on the weight value corresponding to each of the plurality of ROIs and the dose distributions corresponding to the plurality of ROIs. For example, for each of the plurality of ROIs, the processing device 140 may determine whether a product of the dose difference between the planned dose distribution and the predicted dose distribution of the ROI and the weight value corresponding to the ROI is greater than a corresponding dose difference threshold. In response to determining that the product of the dose difference between the planned dose distribution and the predicted dose distribution of the ROI and the weight value corresponding to the ROI is greater than the corresponding dose difference threshold, the processing device 140 may determine that the quality assurance test corresponding to the ROI is not passed. In some embodiments, when the quality assurance tests corresponding to ROIs with relatively high weights are all passed, the processing device 140 may determine that the quality assurance test corresponding to the target plan is passed. In some embodiments, when a count of the ROIs that pass the quality assurance test is greater than a certain count threshold (e.g., 90%, 95%, and 99%), the processing device 140 may determine that the quality assurance test corresponding to the target plan is passed. In some embodiments, when the quality assurance test corresponding to each of the plurality of ROIs is passed, the processing device 140 may determine that the quality assurance test corresponding to the target plan is passed.

In some embodiments, the first threshold, the second threshold, the dose difference threshold, and/or the quantity threshold may be manually set by the user (e.g., the doctor, and the technician) of the medical system 100 or automatically set by the one or more components (e.g., the processing device 140) of the medical system 100 according to different situations.

According to some embodiments of the present disclosure, by determining the predicted dose distribution (e.g., the DVH ) of each ROI in the target subject, the difference between the predicted dose distribution and the planned dose distribution of each ROI in the target subject can be comprehensively and intuitively displayed, making the result of the quality assurance test more clinically meaningful, and reasonable weight values and dose difference thresholds can be set according to the characteristics (e.g., whether the ROI is an eye tissue such as an eye lens, the PTV, or the OAR) of each ROI, thereby improving the rationality and accuracy of the quality assurance test.

In response to determining that the quality assurance test is passed, the process 500 may proceed to operation 560. In operation 560, the processing device 140 (e.g., the control module 440) may perform a target operation on the target subject.

In some embodiments, the target operation may include the following operation. The processing device 140 may control at least one component of the medical device to perform a radiotherapy operation on the target subject based on the values of the radiotherapy parameters of the target plan of the target subject. In some embodiments, the target operation may include the following operation. The user (e.g., the doctor, and the technician) of the medical system 100 may adjust or confirm the target plan according to the prediction result, and the processing device 140 may control the at least one component of the medical device to perform the radiotherapy operation on the target subject based on the adjusted or confirmed values of the radiotherapy parameters of the target plan. In some embodiments, the target operation may include the following operation. The user (e.g., the doctor, and the technician) of the medical system 100 may perform the quality assurance test by executing the values of the radiotherapy parameters of the target plan according to the prediction result using the medical device.

In response to determining that the quality assurance test is not passed, the process 500 may proceed to operation 570. In operation 570, the processing device 140 (e.g., the determination module 430) may adjust the target plan or perform the quality assurance test on the target subject.

In some embodiments, in response to determining that the quality assurance test is not passed, the complexity of the target plan may be appropriately adjusted (e.g., reduced). In some embodiments, in response to determining that the quality assurance test is not passed, the processing device 140 may determine the reason why the quality assurance test is not passed based on the status information of the medical device, the target plan of the target subject, the parameter information of the dose model, and the predicted image using the second model. The processing device 140 may adjust a corresponding parameter value based on the reason why the quality assurance test is not passed. The processing device 140 may determine an updated prediction result based on the adjusted parameter value using the first model. The processing device 140 may determine a target adjustment mode based on the updated prediction result. For example, the processing device 140 may determine whether the quality assurance test is passed based on the updated prediction result. In response to determining that the quality assurance test is passed, the processing device 140 may determine the adjusted parameter value as the target adjustment mode. The processing device 140 may control the medical device to implement the updated target plan on the target subject, the updated target plan being determined based on the adjusted parameter value. More description regarding determining the target adjustment mode may be found elsewhere in the present disclosure (e.g., operations 630-650 in FIG. 6).

In some embodiments, in response to determining that the quality assurance test is not passed, the quality assurance test may be performed on the target subject based on the target plan. For example, the quality assurance test may be performed by executing the values of the radiotherapy parameters of the target plan using the medical device. In response to determining that the quality assurance test is passed, the processing device 140 may control the at least one component of the medical device to perform radiotherapy on the target subject based on the values of the radiotherapy parameters of the target plan for the target subject. In response to determining that the quality assurance test is not passed, the processing device 140 may adjust the target plan until the quality assurance test is passed.

It should be noted that the above description of the present disclosure is provided for the purpose of illustration only and is not intended to limit the scope of the present disclosure. For those of ordinary skill in the art, various changes and modifications can be made according to the description of the present disclosure. However, these changes and modifications do not depart from the scope of the present disclosure. In some embodiments, operations 510-520 may be combined into one operation. In some embodiments, an operation of obtaining the parameter information of the dose model may be added to the process 500, and correspondingly, in operation 540, the processing device 140 may determine the prediction result based on the status information of the medical device, the target plan, and the parameter information of the dose model. In some embodiments, an operation of obtaining the medical image of the target subject may be added to the process 500, and correspondingly, in operation 540, the processing device 140 may determine the prediction result based on the status information of the medical device, the target plan, the parameter information of the dose model, and the medical image of the target subject. In some embodiments, operation 530 may be omitted, and the processing device 140 may directly determine the prediction result based on the status information of the medical device obtained in operation 510 and the target plan obtained in operation 520 without determining whether the status information of the medical device satisfies the preset condition. In some embodiments, the order of the operations in the process 500 may be changed. For example, operation 530 may be performed before operation 520.

In some embodiments, operation 510 and operation 530 may be performed at a certain cycle (e.g., daily, weekly, monthly, and annually) to check whether the status information of the medical device satisfies the requirements. Before the radiotherapy is performed on the target subject, operations 520, 540, and 550 may be performed to determine the prediction result, and whether the quality assurance test is passed may be determined based on the prediction result. In some embodiments, operations 510-550 may be performed before the radiotherapy is performed on the target subject.

FIG. 6 is a flowchart illustrating an exemplary quality assurance process according to some embodiments of the present disclosure. In some embodiments, at least part of a process 600 may be performed by the processing device 140 (e.g., implemented by the computing device 200 shown in FIG. 2). For example, the process 600 may be stored in a storage device (e.g., the storage device 150, the storage device 220, and the storage device 390) in the form of instructions (e.g., application programs) and invoked and/or executed by the processing device 140 (e.g., the processor 210 shown in FIG. 2, the CPU 340 shown in FIG. 3, or the one or more modules of the processing device 140 shown in FIG. 4). The operations of the process shown below are for illustrative purposes only. In some embodiments, the process 600 may be completed using one or more additional operations not described and/or without one or more operations discussed. In addition, the order of the operations of the process 600 shown in FIG. 6 and described below is not intended to be limiting.

In 610, the processing device 140 (e.g., the determination module 430) may determine at least one of a predicted image, a gamma pass rate, or a dose distribution by inputting status information of a medical device and a target plan of a target subject into a first model.

In some embodiments, the processing device 140 may determine a first eigenvector based on the status information of the medical device. For example, the processing device 140 may use a systematic error and a random error of at least one component of the medical device as eigenvalues of the first eigenvector. As another example, the processing device 140 may use beam and dose information (e.g., beam flatness, beam symmetry, beam linearity, etc.), positioning accuracy information of the at least one component (e.g., a gantry, a scanning table, a radiotherapy radiation source, and a beam limiter) of the medical device, an operation error of at least one component of the medical device, and a data set execution result of the medical device as the eigenvalues of the first eigenvector. The processing device 140 may determine a second eigenvector based on the target plan of the target subject. For example, the processing device 140 may use a feature related to the complexity of the target plan and a target flux map as eigenvalues of the second eigenvector. The processing device 140 may input the first eigenvector and the second eigenvector into the first model, and the first model may output at least one of the predicted image, the gamma pass rate, or the dose distribution. For example, the first model may output the predicted image (e.g., a predicted E PID image), and the processing device 140 may determine the gamma pass rate through gamma analysis based on the predicted image and a calculated image (e.g., a calculated EPID image). The calculated image (e.g., the calculated EPID image) may be an EPID image determined by the medical system 100 (e.g., a treatment planning system (TPS) of the medical system 100) based on the target plan of the target subject. As another example, the first model may directly output the gamma pass rate. In some embodiments, the processing device 140 may input the first eigenvector and the second eigenvector into the first model, and the first model may output the dose distribution in the target subject. For example, the first model may output a DVH. In some embodiments, the processing device 140 may determine one or more eigenvectors based on a medical image of the target subject and/or parameter information of a dose model, and use the one or more eigenvectors as input information of the first model.

In some embodiments, the processing device 140 may input a first target flux map into a first feature extraction model (e.g., a two-dimensional feature extraction model). In this embodiment, the first target flux map may be determined based on a medical device without the systematic error and/or the random error. The first feature extraction model may determine a two-dimensional eigenvector corresponding to the first target flux map. The processing device 140 may input the systematic error and/or the random error of the at least one component of the medical device into a second feature extraction model (e.g., a one-dimensional feature extraction model), and the second feature extraction model may determine a one-dimensional eigenvector corresponding to the systematic error and/or the random error. The processing device 140 may input the one-dimensional eigenvector corresponding to the systematic error and/or the random error into a dimension conversion model, and the dimension conversion model may convert the one-dimensional eigenvector corresponding to the systematic error and/or the random error into a two-dimensional eigenvector corresponding to the systematic error and/or the random error. Further, the processing device 140 may input the two-dimensional eigenvector corresponding to the first target flux map and the two-dimensional eigenvector corresponding to the systematic error and/or the random error into the first model, and the first model may output the predicted image. The first feature extraction model and the second feature extraction model refer to machine learning models or algorithms for converting the input information of the model into an eigenvector. The dimension conversion model refers to a machine learning model or an algorithm for converting a data dimension (e.g., conversion among the one-dimensional eigenvector, the two-dimensional eigenvector, and a three-dimensional eigenvector).

In some embodiments, the processing device 140 may input a second target flux map into the first feature extraction model (e.g., the two-dimensional feature extraction model). In this embodiment, the second target flux map may be determined based on the medical device with the systematic error and/or the random error. The first feature extraction model may determine the two-dimensional eigenvector corresponding to the second target flux map. Further, the processing device 140 may input the two-dimensional eigenvector corresponding to the second target flux map into the first model, and the first model may output the predicted image.

In some embodiments, the processing device 140 may input the first target flux map into the first feature extraction model (e.g., the two-dimensional feature extraction model). In this embodiment, the first target flux map may be determined based on the medical device without the systematic error and/or the random error. The first feature extraction model may determine the two-dimensional eigenvector corresponding to the first target flux map. The processing device 140 may input the two-dimensional eigenvector corresponding to the first target flux map into the dimension conversion model, and the dimension conversion model may convert the two-dimensional eigenvector corresponding to the first target flux map into the one-dimensional eigenvector corresponding to the first target flux map. Then the processing device 140 may input the systematic error and/or the random error of the at least one component of the medical device, the feature related to the complexity of the target plan, the parameter information of the dose model, or the like, or any combination thereof into the second feature extraction model (e.g., the one-dimensional feature extraction model). The second feature extraction model may determine the one-dimensional eigenvector corresponding to the systematic error and/or the random error, the one-dimensional eigenvector corresponding to the complexity of the target plan, the one-dimensional eigenvector corresponding to the parameter information of the dose model, or the like, or any combination thereof. Further, the processing device 140 may input the one-dimensional eigenvector corresponding to the first target flux map, the one-dimensional eigenvector corresponding to the systematic error and/or the random error, the one-dimensional eigenvector corresponding to the complexity of the target plan, the one-dimensional eigenvector corresponding to the parameter information of the dose model, or the like, or any combination thereof into the first model, and the first model may output the gamma pass rate.

In the present disclosure, model input data (e.g., the status information of the medical device, the target plan of the target subject, and the parameter information of the dose model) in operation 610 is also referred to as initial model input data (e.g., initial status information of the medical device, an initial target plan, and parameter information of an initial dose model), and the prediction result (e.g., the predicted image, the gamma pass rate, and the dose distribution) determined in operation 610 is also referred to as an initial prediction result (e.g., an initial predicted image, an initial gamma pass rate, and an initial dose distribution).

In 620, the processing device 140 (e.g., the determination module 430) may determine whether a quality assurance test is passed based on at least one of the predicted image, the gamma pass rate, or the dose distribution.

For example, the processing device 140 may determine whether the quality assurance test is passed based on the gamma pass rate. As another example, the processing device 140 may determine whether the quality assurance test is passed based on a difference between the predicted image and the calculated image. As another example, the processing device 140 may determine whether the quality assurance test is passed based on a difference between a planned dose distribution and a predicted dose distribution. More descriptions regarding determining whether the quality assurance test is passed may be found elsewhere in the present disclosure (e.g., operation 550 in FIG. 5).

In some embodiments, in response to determining that the quality assurance test is passed, the processing device 140 may control the medical device to perform the radiotherapy on the target subject based on the target plan.

In 630, in response to determining that the quality assurance test is not passed, the processing device 140 (e.g., the determination module 430) may determine the reason why the quality assurance test is not passed based on the status information of the medical device, the target plan of the target subject, parameter information of a dose model, and the predicted image using a second model.

In some embodiments, the processing device 140 may determine the first eigenvector based on the status information of the medical device. The processing device 140 may determine the second eigenvector based on the target plan of the target subject. The processing device 140 may determine a third eigenvector based on the parameter information of the dose model. The processing device 140 may determine a fourth eigenvector based on the predicted image. The processing device 140 may determine the reason why the quality assurance test is not passed based on at least one of the first eigenvector, the second eigenvector, the third eigenvector, and the fourth eigenvector using the second model. For example, the processing device 140 may input the first eigenvector, the second eigenvector, the third eigenvector, and the fourth eigenvector into the second model, and the second model may output the reason why the quality assurance test is not passed. As another example, the processing device 140 may input the first eigenvector, the second eigenvector, and the fourth eigenvector into the second model, and the second model may output the reason why the quality assurance test is not passed.

For example, the processing device 140 may input the first target flux map into the first feature extraction model (e.g., the two-dimensional feature extraction model). In this embodiment, the first target flux map may be determined based on the medical device without the systematic error and/or the random error. The first feature extraction model may determine the two-dimensional eigenvector corresponding to the first target flux map. The processing device 140 may input the two-dimensional eigenvector corresponding to the first target flux map into the dimension conversion model, and the dimension conversion model may convert the two-dimensional eigenvector corresponding to the first target flux map into the one-dimensional eigenvector corresponding to the first target flux map. Then the processing device 140 may input the systematic error and/or the random error of the at least one component of the medical device, the feature related to the complexity of the target plan, and the parameter information of the dose model into the second feature extraction model (e.g., the one-dimensional feature extraction model), and the second feature extraction model may determine the one-dimensional eigenvector corresponding to the systematic error and/or the random error, the one-dimensional eigenvector corresponding to the complexity of the target plan, and the one-dimensional eigenvector corresponding to the parameter information of the dose model. Further, the processing device 140 may input the one-dimensional eigenvector corresponding to the first target flux map, the one-dimensional eigenvector corresponding to the systematic error and/or the random error, the one-dimensional eigenvector corresponding to the complexity of the target plan, and the one-dimensional eigenvector corresponding to the parameter information of the dose model into the second model, and the second model may output the reason why the quality assurance test is not passed.

The reason why the quality assurance test is not passed may include a reason related to the medical device, a reason related to the target plan of the target subject, a reason related to parameters of the dose model, or the like, or any combination thereof. The reason related to the medical device may include a large operation error of the medical device (e.g., a large systematic error and random error of a multi-leaf collimator). The reason related to the target plan of the target subject may include the high complexity of the target plan (e.g., a large or small area of the radiation field, the high degree of deviation of the radiation field from an isocenter point of the medical device). The reason related to the parameters of the dose model may include an inappropriate parameter of the dose model, etc.

In some embodiments, the second model may output a plurality of reasons why the quality assurance test is not passed and prioritize the plurality of reasons why the quality assurance test is not passed. For example, the plurality of reasons may be prioritized in a descending order according to the possibilities of causing the quality assurance test to be not passed.

In 640, the processing device 140 (e.g., the determination module 430) may adjust a corresponding parameter value based on the reason why the quality assurance test is not passed.

For example, if the reason why the quality assurance test is not passed is the reason related to the medical device, the processing device 140 or the user (e.g., a physicist or the technician) may adjust an operation parameter (e.g., improve an operation accuracy of the component) of the at least one component of the medical device. As another example, if the reason why the quality assurance test is not passed is the reason related to the target plan, the processing device 140 or the user (e.g., the doctor) may adjust the values of the radiotherapy parameters of the target plan (e.g., increase or decrease the values of the radiotherapy parameters) to reduce the complexity of the target plan. As another example, if the reason why the quality assurance test is not passed is the reason related to the parameters of the dose model, the processing device 140 or the user (e.g., the physicist or the technician) may adjust the values of the parameters of the dose model (e.g., use another type of dose model, and increase or decrease the values of the parameters of the dose model).

In some embodiments, when there are a plurality of reasons why the quality assurance test is not passed, the processing device 140 or the user (e.g., the doctor) may adjust a parameter value corresponding to each of the plurality of reasons why the quality assurance test is not passed, or adjust the parameter values corresponding to at least two of the plurality of reasons why the quality assurance test is not passed simultaneously.

In 650, the processing device 140 (e.g., the determination module 430) may determine an updated prediction result based on the adjusted parameter value using the first model.

In some embodiments, the processing device 140 may input the adjusted parameter value and other initial model input data (i.e., other unadjusted initial model input data) into the first model, and the first model may output an updated predicted image, an updated dose distribution, and/or an updated gamma pass rate. For example, if the reason why the quality assurance test is not passed is the reason related to the target plan, after the values of the radiotherapy parameters of the target plan are adjusted, the processing device 140 may input the adjusted target plan and the initial status information of the medical device into the first model, and the first model may output the updated predicted image, the updated dose distribution, and/or the updated gamma pass rate. As another example, if the reason why the quality assurance test is not passed is the reason related to the medical device, after the operation accuracy of the at least one component of the medical device is improved, the processing device 140 may input the adjusted status information of the medical device and the initial target plan into the first model, and the first model may output the updated predicted image, the updated dose distribution, and/or the updated gamma pass rate.

In 660, the processing device 140 (e.g., the determination module 430) may determine a target adjustment mode based on the updated prediction result.

In some embodiments, if the updated prediction result is better than the initial prediction result (e.g., the updated gamma pass rate is higher than the initial gamma pass rate determined in operation 610), the processing device 140 may determine a current parameter adjustment mode (i.e., the parameter adjustment mode in operation 640) as the target adjustment mode. For example, assuming that the reason why the quality assurance test is not passed is the reason related to the target plan, the processing device 140 may reduce the area of the radiation field in the target plan, and input the reduced area of the radiation field and other initial model input data (e.g., the initial status information of the medical device, and the parameter information of the initial dose model) into the first model, and the first model may output the updated gamma pass rate. If the updated gamma pass rate is higher than the initial gamma pass rate, the processing device 140 may determine that the target adjustment mode is to reduce the area of the radiation field. In some embodiments, if the updated gamma pass rate is lower than the initial gamma pass rate, the processing device 140 may determine that the target adjustment mode is to increase the area of the radiation field.

In some embodiments, if the initial prediction result is better than the updated prediction result (e.g., the updated gamma pass rate is lower than the initial gamma pass rate determined in operation 610), the processing device 140 may adjust other parameter values (e.g., other parameter values corresponding to the reason why the quality assurance test is not passed or parameter values corresponding to other priorities of reasons why the quality assurance test is not passed) based on the reason why the quality assurance test is not passed, as described in operation 640. The processing device 140 may determine a second updated prediction result based on the adjusted parameter value using the first model, as described in operation 650. The processing device 140 may determine the target adjustment mode based on the second updated prediction result. Operations 640-660 may be repeatedly performed until the updated prediction result is better than the initial prediction result, and the processing device 140 may determine the current parameter adjustment mode as the target adjustment mode.

In some embodiments, the processing device 140 may determine whether the quality assurance test is passed based on the updated prediction result. In response to determining that the quality assurance test is passed, the processing device 140 may determine the current parameter adjustment mode (i.e., the parameter adjustment mode in operation 640) as the target adjustment mode. The processing device 140 may control the medical device to execute the updated target plan on the target subject. The updated target plan may be determined based on the adjusted parameter value.

In some embodiments, the processing device 140 may send a reminder to the user. The reminder may be in the form of a text prompt, a voice prompt, an image prompt, a video prompt, an indicator light reminder, or the like, or any combination thereof. In some embodiments, the reminder may include the result (e.g., passed or not passed) of the quality assurance test. In some embodiments, if the quality assurance test is not passed, the reminder may include the reason why the quality assurance test is not passed and/or the target adjustment mode.

It should be noted that the above description of the present disclosure is provided for the purpose of illustration only and is not intended to limit the scope of the present disclosure. For those of ordinary skill in the art, various changes and modifications can be made according to the description of the present disclosure. However, these changes and modifications do not depart from the scope of the present disclosure. In some embodiments, the first model and the second model may be used in combination. For example, an output layer of the first model may be connected with an input layer of the second model. The processing device 140 may input the status information of the medical device, the target plan of the target subject, the medical image of the target subject, and the parameter information of the dose model into the input layer of the first model, and the predicted image, the dose distribution, and/or the gamma pass rate output by the first model may be directly input into the input layer of the second model. The second model may output the predicted image, the dose distribution, the gamma pass rate, and the reason why the quality assurance test is not passed.

FIG. 7 is a flowchart illustrating an exemplary process of joint training of a first model and a second model according to some embodiments of the present disclosure. In some embodiments, at least part of a process 700 may be performed by the processing device 140 (e.g., implemented by the computing device 200 shown in FIG. 2). For example, the process 700 may be stored in a storage device (e.g., the storage device 150, the storage device 220, and the storage device 390) in the form of instructions (e.g., application programs) and invoked and/or executed by the processing device 140 (e.g., the processor 210 shown in FIG. 2, the CPU 340 shown in FIG. 3, or the one or more modules of the processing device 140 shown in FIG. 4). The operations of the process shown below are for illustrative purposes only. In some embodiments, the process 700 may be completed using one or more additional operations not described and/or without one or more operations discussed. In addition, the order of the operations of the process 700 shown in FIG. 7 and described below is not intended to be limiting.

In 710, the processing device 140 (e.g., the determination module 430) may obtain a plurality sets of training samples.

Each set of the plurality sets of training samples may include sample status information of a medical device, a sample plan, sample parameter information of a dose model, a reference image, and a reference reason why a quality assurance test is not passed. The reference image and the reference reason why the quality assurance test is not passed may be used as gold standards for model training. The sample status information of the medical device may include beam and dose information, positioning accuracy information of at least one component of the medical device, an operation error (e.g., a systematic error, and a random error) of at least one component of the medical device, a data set execution result using the medical device, or the like, or any combination thereof, as described in operation 510. The sample plan may include radiotherapy parameters involved in a radiotherapy process, a sample flux map, and a feature related to the complexity of the radiotherapy plan, as described in operation 520. The sample parameter information of the dose model may include a type of the dose model (e.g., a type of a dose algorithm), parameters of the dose model (e.g., parameters of the dose algorithm), etc., as described in operation 510.

In some embodiments, the plurality sets of training samples may include historical data related to a historical scanning process. For example, the plurality sets of training samples may include historical status information of the medical device, historical radiotherapy plans, historical parameter information of the dose model, historical images, and historical reasons why the quality assurance test is not passed related to historical radiotherapy processes of a plurality of candidate subjects (e.g., patients).

In some embodiments, the plurality sets of training samples may include simulation data. A user of the processing device 140 or the medical system 100 may generate the simulation data by adjusting the historical data related to the historical scanning process. For example, the sample plan may be determined by adjusting the values of the radiotherapy parameters of the historical radiotherapy plan to obtain the sample plan of an expected complexity (e.g., a relatively high complexity). By using the simulation data as the training samples, the diversity of the training samples may be increased, making the training samples richer, thereby improving the accuracy and effectiveness of the model trained based on the training samples.

In 720, the processing device 140 (e.g., the determination module 430) may generate a candidate image by inputting the sample status information of the medical device and the sample plan of the plurality sets of training samples into a first initial sub-model.

In some embodiments, one or more parameter values of a first initial sub-model (and/or a second initial sub-model) may be iteratively updated by performing multiple iterations. Exemplary parameters of the first initial sub-model (and/or the second initial sub-model) may include a size of a kernel of a layer, a total count of layers, a count of nodes in each layer, a connection weight between two connected nodes, a deviation vector related to a node, or the like, or any combination thereof. In each iteration, the processing device 140 may obtain a set of training samples from the plurality sets of training samples to jointly train the first initial sub-model (and/or the second initial sub-model). A certain iteration is illustrated as an example in the following.

The first initial sub-model refers to a machine learning model that needs to be trained to generate a first model. In some embodiments, the processing device 140 may input the sample status information the medical device and the sample plan in a certain set of training samples of the plurality sets of training samples into an input layer of the first initial sub-model, and the first initial sub-model may determine a first predicted output (i.e., a candidate image) based on the sample status information of the medical device and the sample plan.

In 730, the processing device 140 (e.g., the determination module 430) may generate a candidate reason why the quality assurance test is not passed by inputting the sample status information of the medical device, the sample plan, the sample parameter information of the dose model, and the candidate image into a second initial sub-model.

The second initial sub-model refers to a machine learning model that needs to be trained to generate a second model. In some embodiments, the sample status information of the medical device, the sample plan, the sample parameter information of the dose model in the training samples, and the candidate image output by the first initial sub-model may be input into an input layer of the second initial sub-model, and the second initial sub-model may determine a second predicted output (i.e., the candidate reason why the quality assurance test is not passed) based on the sample status information of the medical device, the sample plan, the sample parameter information of the dose model, and the candidate image.

In 740, the processing device 140 (e.g., the determination module 430) may determine a value of a first loss function based on the candidate image and the reference image.

The first loss function may be configured to evaluate a difference between the predicted output (e.g., the candidate image) and an expected output (e.g., the reference image) of the first initial sub- model. In some embodiments, if the value of the first loss function is greater than a first threshold in the current iteration, a parameter value of the first initial sub-model may be adjusted to reduce the value of the first loss function (i.e., reduce the difference between the candidate image and the reference image).

In 750, the processing device 140 (e.g., the determination module 430) may determine a value of a second loss function based on the candidate reason why the quality assurance test is not passed and the reference reason why the quality assurance test is not passed.

The second loss function may be configured to evaluate a difference between the predicted output (e.g., the candidate reason why the quality assurance test is not passed) and the expected output (e.g., the reference reason why the quality assurance test is not passed) of the second initial sub-model. In some embodiments, if the value of the second loss function is greater than a second threshold in the current iteration, a parameter value of the second initial sub-model may be adjusted to reduce the value of the second loss function (i.e., reduce the difference between the candidate reason why the quality assurance test is not passed and the reference reason why the quality assurance test is not passed).

In 760, the processing device 140 (e.g., the determination module 430) may determine a value of a target loss function based on the value of the first loss function and the value of the second loss function.

In some embodiments, the processing device 140 may obtain a first weight corresponding to the first loss function and a second weight corresponding to the second loss function. The first weight and the second weight respectively indicate the importance of the first loss function and the second loss function when the first initial sub-model and the second initial sub-model are jointly trained based on the target loss function. The processing device 140 may determine the value of the target loss function based on the value of the first loss function, the first weight corresponding to the first loss function, the value of the second loss function, and the second weight corresponding to the second loss function. For example, assuming that the value of the first loss function is A1, the value of the second loss function is A2, the first weight corresponding to the first loss function is W1, and the second weight corresponding to the second loss function is W2, the value of the target function is denoted as A=A1 × W1 + A2 × W2. In some embodiments, the first weight and the second weight may be manually set by the user (e.g., the doctor, and the technician) of the medical system 100 or automatically set by one or more components (e.g., the processing device 140) of the medical system 100 according to different situations.

In 770, the processing device 140 (e.g., the determination module 430) may update at least one of the first initial sub-model and the second initial sub-model by optimizing the value of the target loss function to generate a first model and a second model.

In some embodiments, if the value of the target loss function is greater than a third threshold in the current iteration, the processing device 140 may adjust the parameter values of the first initial sub-model and/or the second initial sub-model to reduce the value of the target loss function. In some embodiments, the parameter values of the first initial sub-model and/or the second initial sub-model may be updated by performing multiple iterations until the value of the target loss function is less than or equal to the third threshold. If the value of the target loss function is less than or equal to the third threshold in the current iteration, the processing device 140 may use an updated first initial sub-model and an updated second initial sub-model generated in the current iteration as a trained first model and a trained second model.

In some embodiments, if the value of the target loss function converges, the updated first initial sub-model and the updated second initial sub-model generated in the current iteration may be used as the trained first model and the trained second model. If the change in the value of the target loss function in two or more consecutive iterations is less than a fourth threshold, the value of the target loss function is considered to be converged. In some embodiments, when a count of iterations performed during the training process is greater than a fifth threshold, the updated first initial sub-model and the updated second initial sub-model generated in the current iteration may be used as the trained first model and the trained second model.

In some embodiments, the first threshold, the second threshold, the third threshold, the fourth threshold, and/or the fifth threshold may be manually set by the user (e.g., the doctor, and the technician) of the medical system 100 or automatically set by the one or more components (e.g., the processing device 140) of the medical system 100 according to different situations.

It should be noted that the above description of the present disclosure is provided for illustrative purposes only and is not intended to limit the scope of the present disclosure. For those of ordinary skill in the art, various changes and modifications can be made according to the description of the present disclosure. However, these changes and modifications do not depart from the scope of the present disclosure. In some embodiments, in the joint training of the process 700 of the first initial sub-model and the second initial sub-model, the operation of determining the value of the first loss function may be omitted, i.e., the value of the target loss function may be the value of the second loss function, and the processing device 140 may perform joint training on the first initial sub-model and the second initial sub-model based on the value of the second loss function.

In some embodiments, the processing device 140 may train the first initial sub-model and the second initial sub-model separately to generate the first model and the second model, respectively. Taking the process of training the first model as an example, the processing device 140 may obtain a plurality sets of first training samples. Each set of the plurality sets of first training samples may include the sample status information of the medical device, the sample plan, and a reference prediction result. The reference prediction result may include a reference image and/or a reference gamma pass rate. The processing device 140 may generate the first model by training the first initial sub-model based on the plurality sets of first training samples, and the training process may include multiple iterations. In the first iteration, the processing device 140 may obtain the first initial sub-model. In other iterations, the processing device 140 may obtain a first updated sub-model generated in the previous iteration. The processing device 140 may input the sample status information of the medical device and the sample plan in the first training samples into an input layer of the first initial sub-model (or the first updated sub-model), and input the reference prediction result in the first training samples into an output layer of the first initial sub-model (or the first updated sub-model). The first initial sub-model (or the first updated sub-model) may output a candidate prediction result. The candidate prediction result may include a candidate image and/or a candidate gamma pass rate. The candidate prediction result may be compared with the reference prediction result based on a loss function. In some embodiments, the parameter value of the first initial sub-model may be updated by performing multiple iterations until a termination condition is satisfied. The termination condition may be related to the loss function or the count of iterations in the iterative process. For example, if the value of the loss function is less than a threshold, it is determined that the termination condition is satisfied. As another example, if the value of the loss function converges, it is determined that the termination condition is satisfied. As another example, when a specified number of iterations are performed during the training process, it is determined that the termination condition is satisfied. If the current iteration satisfies the termination condition, the first updated sub-model generated in the current iteration may be determined as the first model. If the current iteration does not satisfy the termination condition, the parameter value of the first updated sub-model generated in the current iteration may be adjusted until the iteration satisfies the termination condition. Similarly, in the process of training the second model, the processing device 140 may obtain the plurality sets of second training samples. Each set of the plurality sets of second training samples may include the sample status information of the medical device, the sample plan, the sample parameter information of the dose model, the sample image, and the reference prediction result. The reference prediction result may include the reason why the quality assurance test is not passed. The processing device 140 may generate the second model by training the second initial sub-model based on the plurality sets of second training samples. It should be noted that the above description of model training is provided for illustrative purposes only and is not intended to limit the scope of the present disclosure. The first training samples and the second training samples may also include other information or may not include the above information. For example, the first training samples may also include sample parameter information of the dose model and/or a sample medical image. As another example, the first training samples and/or the second training samples may include a feature related to the complexity of the target plan and/or a target flux map. As another example, the second training sample may not include the sample parameter information of the dose model. As another example, the dose assurance model may include only the first model, and in this case, the first model may be generated by only training the first initial sub-model.

In some embodiments, the processing device 1 40 may first train the first initial sub-model and the second initial sub-model separately to generate the first candidate model and the second candidate model, respectively. Then, the processing device 140 may jointly train the first candidate model and the second candidate model to generate the first model and the second model. In some embodiments, the processing device 140 may first jointly train the first initial sub-model and the second initial sub-model to generate the first candidate model and the second candidate model, respectively. Then, the processing device 140 may separately train the first candidate model and the second candidate model to generate the first model and the second model.

In some embodiments, a quality assurance model (e.g., the first model, and the second model) may be updated periodically or aperiodically. For example, the quality assurance model may be updated based on new training samples. In some embodiments, the generation, training, and/or updating of the quality assurance model may be performed online in response to a request for the quality assurance test. In some embodiments, the generation, training, and/or updating of the quality assurance model may be performed offline.

In some embodiments, the generation, training, and/or updating of the quality assurance model (e.g., the first model, and the second model) may be performed on a first system, and the quality assurance test based on the quality assurance model may be performed on a second system. The first system and the second system may be the same or different. The first system and/or the second system may include the medical system 100. For example, the generation, training, and/or updating of the quality assurance model may be performed on a first processing device of the medical system 100, and the application of the quality assurance model may be performed on a second processing device of the medical system 100. The first processing device and the second processing device may be the same or different. The first processing device and/or the second processing device may include the processing device 140.

Compared with the prior art, the beneficial effects that may be brought about by the above embodiments of the present disclosure include but are not limited to: (1) The operation status of the medical device changes as the use time of the medical device increases. Different operation statuses of the medical device have a great impact on the result of the quality assurance test. When the quality assurance test is performed, the current operation error of the medical device is fully considered, which can make the result of the quality assurance test more accurate and effective. (2) The data set execution results obtained by executing different types of data sets (e.g., the first data set, the second data set, and the third data set) using the medical device can reflect the accuracy of the medical device in executing different types of radiotherapy plans, such that the execution effect of the medical device in executing the radiotherapy plan can be determined without using the radiotherapy plan for a specific patient. (3) When the dose calculation is performed, the medical device is modeled to improve the efficiency of dose calculation, and different dose models and different dose model parameters have a great impact on the result of the quality assurance test. When the quality assurance test is performed, the parameter information of the dose model is fully considered, which can make the result of the quality assurance test more accurate and effective. (4) The prediction result is determined by the machine learning method based on one or more of the status information of the medical device, the target plan of the target subject, and the parameter information of the dose model, which can save the time of the quality assurance test, improve the efficiency of the quality assurance test, and reduce the labor intensity of the operator. (5) The reason why the quality assurance test is not passed can be analyzed through the result of the quality assurance test, and the recommended adjustment mode can be further provided, thereby saving the time of the operator, and improving the efficiency of the radiotherapy process. (6) The quality assurance model can be updated and trained based on different training samples. For example, the quality assurance model can be updated and trained with the development of radiotherapy technology and changes in application scenarios, so as to ensure the rationality and accuracy of the quality assurance test based on the quality assurance model. It should be noted that different embodiments may produce different beneficial effects. In different embodiments, the beneficial effects that may be produced may be any one or any combination of the above, or any other beneficial effects that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

In addition, it will be understood by those having ordinary skill in the art that various aspects of present disclosure may be illustrated and described by a number of patentable categories or situations, including any new and useful process, machine, product, or combination of substances, or any new and useful improvement thereof. Therefore, various aspects of the present disclosure may be implemented entirely by hardware, entirely by software (including firmware, resident software, microcode, etc.), or by a combination of hardware and software. The above hardware or software may be referred to as a "unit," "module," or "system." In addition, various aspects of present disclosure may take the form of a computer program product embodied in one or more computer-readable media, wherein computer-readable program codes are contained therein.

A computer-readable signal medium may include a propagated data signal containing computer program codes, for example, in baseband or as part of a carrier wave. Such propagated signals may be in various forms, including electromagnetic, optical, or the like, or any suitable combination thereof. The computer-readable signal medium may be any computer readable medium other than a computer-readable storage medium, which may be connected to an instruction execution system, device or apparatus to communicate, propagate or transmit a program for use. The program codes on the computer-readable signal medium may be propagated via any suitable medium, including a radio, a cable, a fiber optic cable, RF, or the like, or any combination thereof.

The computer program codes required for the operation of each part of the present disclosure can be written in any one or more programming languages, including object-oriented programming languages such as Java, Scala, Smalltalk, Eiffel, JADE, Emerald, C++, C#, VB.NET, Python, etc., conventional procedural programming languages such as C programming language, VisualBasic, Fortran2103, Perl, COBOL2102, PHP, ABAP, dynamic programming languages such as Python, Ruby and Groovy, or other programming languages. The program codes can be run entirely on the user's computer, or run on the user's computer as an independent software package, or run partially on the user's computer and partially on a remote computer, or run entirely on a remote computer or server. In the latter case, the remote computer can be connected to the user's computer through any type of network (including a local area network (LAN) or a wide area network (WAN)), or can establish a connection with an external computer (for example, by using the network of a network service provider) or in a cloud computing environment or as a service, for example, software as a service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about," "approximately," or "substantially" in some examples. Unless otherwise stated, "about," "approximately," or "substantially" indicates that the number is allowed to vary by ±20%. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A quality assurance method, comprising:
obtaining status information of a medical device;
obtaining a target plan of a target subject;
determining a prediction result based on the status information of the medical device and the target plan; and
determining whether a quality assurance test is passed based on the prediction result.

2. The quality assurance method of claim 1, wherein the status information of the medical device includes at least one of beam information corresponding to a plurality of dose rates, positioning accuracy information of at least one component of the medical device corresponding to a plurality of positions, or an operation error of at least one component of the medical device.

3. The quality assurance method of claim 1 or 2, wherein the obtaining status information of a medical device includes:
obtaining at least one data set, wherein the at least one data set is determined based on at least one of a candidate plan, a limit performance of the at least one component of the medical device, or a calculation limit of a dose model; and
determining the status information of the medical device by implementing the at least one data set using the medical device.

4. The quality assurance method of claim 3, wherein the determining a prediction result based on the status information of the medical device and the target plan includes:
determining whether the status information of the medical device satisfies a preset condition; and
in response to determining that the status information of the medical device satisfies the preset condition, determining the prediction result based on the status information of the medical device and the target plan.

5. The quality assurance method of claim 4, wherein the at least one data set includes at least one of a first data set, a second data set, or a third data set, and the obtaining at least one data set includes:
determining the first data set based on at least one first candidate plan, wherein a value of a first candidate parameter of the at least one first candidate plan is within a first preset range;
determining the second data set based on at least one second candidate plan, wherein a value of a second candidate parameter of the at least one second candidate plan is outside a second preset range; and
determining the third data set based on the limit performance of the at least one component of the medical device and/or the calculation limit of the dose model.

6. The quality assurance method of claim 5, wherein the at least one data set is the second data set or the third data set, and the determining whether the status information of the medical device satisfies a preset condition includes:
determining a plurality of test results by performing a plurality of tests based on the at least one data set; and
determining whether the status information of the medical device satisfies the preset condition based on the plurality of test results.

7. The quality assurance method of any one of claims 1-6, wherein the obtaining a target plan of a target subject includes:
determining a feature associated with a complexity of the target plan and/or a target flux map based on the target plan.

8. The quality assurance method of any one of claims 1-7, wherein the determining a prediction result based on the status information of the medical device and the target plan includes:
determining the prediction result based on the status information of the medical device and the target plan of the target subject using a quality assurance model, wherein the quality assurance model is a machine learning model.

9. The quality assurance method of claim 8, wherein the quality assurance model includes a first model, and the determining the prediction result based on the status information of the medical device and the target plan of the target subject using a quality assurance model includes:
determining the prediction result by inputting the status information of the medical device and the target plan of the target subject into the first model, wherein the prediction result includes at least one of a predicted image, a gamma pass rate, or a dose distribution.

10. The quality assurance method of claim 9, wherein the quality assurance model includes a second model, and the quality assurance method further comprises:
in response to determining that the quality assurance test is not passed, determining a reason why the quality assurance test is not passed based on the status information of the medical device, the target plan of the target subject, and the prediction result using the second model.

11. The quality assurance method of claim 10, further comprising:
obtaining an adjusted parameter value by adjusting a parameter value based on the reason why the quality assurance test is not passed;
determining an updated prediction result based on the adjusted parameter value using the first model;
determining whether the quality assurance test is passed based on the updated prediction result; and
in response to determining that the quality assurance test is passed, controlling the medical device to implement an updated target plan on the target subject, wherein the updated target plan is determined based on the adjusted parameter value.

12. The quality assurance method of any one of claims 8-11, wherein the determining the prediction result based on the status information of the medical device and the target plan of the target subject using a quality assurance model includes:
obtaining an image of the target subject; and
determining a three-dimensional dose distribution in the target subject based on the status information of the medical device, the target plan of the target subject, and the image of the target subject using the quality assurance model.

13. The quality assurance method of any one of claims 1-12, wherein the target subject includes a plurality of regions of interest (ROI), the prediction result includes dose distributions corresponding to the plurality of ROIs, and the determining whether the quality assurance test is passed based on the prediction result includes:
obtaining a weight value corresponding to each of the plurality of ROIs; and
determining whether the quality assurance test is passed based on the weight value corresponding to each of the plurality of ROIs and the dose distributions corresponding to the plurality of ROIs.

14. A quality assurance system, comprising:
at least one storage device configured to store an instruction set; and
at least one processor in communication with the at least one storage device, wherein when executing the instruction set, the at least one processor is configured to cause the system to:
obtain status information of a medical device;
obtain a target plan of a target subject;
determine a prediction result based on the status information of the medical device and the target plan; and
determine whether a quality assurance test is passed based on the prediction result.

15. The quality assurance system of claim 14, wherein the status information of the medical device includes at least one of beam information corresponding to a plurality of dose rates, positioning accuracy information of at least one component of the medical device corresponding to a plurality of positions, or an operation error of at least one component of the medical device.

16. The quality assurance system of claim 14 or 15, wherein the obtaining status information of a medical device includes:
obtaining at least one data set, wherein the at least one data set is determined based on at least one of a candidate plan, a limit performance of the at least one component of the medical device, or a calculation limit of a dose model; and
determining the status information of the medical device by implementing the at least one data set using the medical device.

17. The quality assurance system of claim 16, wherein the determining a prediction result based on the status information of the medical device and the target plan includes:
determining whether the status information of the medical device satisfies a preset condition; and
in response to determining that the status information of the medical device satisfies the preset condition, determining the prediction result based on the status information of the medical device and the target plan.

18. The quality assurance system of claim 17, wherein the at least one data set includes at least one of a first data set, a second data set, or a third data set, and the obtaining at least one data set includes:
determining the first data set based on at least one first candidate plan, wherein a value of a first candidate parameter of the at least one first candidate plan is within a first preset range;
determining the second data set based on at least one second candidate plan, wherein a value of a second candidate parameter of the at least one second candidate plan is outside a second preset range; and
determining the third data set based on the limit performance of the at least one component of the medical device and/or the calculation limit of the dose model.

19. The quality assurance system of claim 18, wherein the at least one data set is the second data set or the third data set, and the determining whether the status information of the medical device satisfies a preset condition includes:
determining a plurality of test results by performing a plurality of tests based on the at least one data set; and
determining whether the status information of the medical device satisfies the preset condition based on the plurality of test results.

20. The quality assurance system of any one of claims 14-19, wherein the obtaining a target plan of a target subject includes:
determining a feature associated with a complexity of the target plan and/or a target flux map based on the target plan.

21. The quality assurance system of any one of claims 14-20, wherein the determining a prediction result based on the status information of the medical device and the target plan includes:
determining the prediction result based on the status information of the medical device and the target plan of the target subject using a quality assurance model, wherein the quality assurance model is a machine learning model.

22. The quality assurance system of claim 21, wherein the quality assurance model includes a first model, and the determining the prediction result based on the status information of the medical device and the target plan of the target subject using a quality assurance model includes:
determining the prediction result by inputting the status information of the medical device and the target plan of the target subject into the first model, wherein the prediction result includes at least one of a predicted image, a gamma pass rate, or a dose distribution.

23. The quality assurance system of claim 22, wherein the quality assurance model includes a second model, and the at least one processor is configured to cause the system to:
in response to determining that the quality assurance test is not passed, determine a reason why the quality assurance test is not passed based on the status information of the medical device, the target plan of the target subject, and the prediction result using the second model.

24. The quality assurance system of claim 23, wherein the at least one processor is configured to cause the system to:
obtain an adjusted parameter value by adjusting a parameter value based on the reason why the quality assurance test is not passed;
determine an updated prediction result based on the adjusted parameter value using the first model;
determine whether the quality assurance test is passed based on the updated prediction result; and
in response to determining that the quality assurance test is passed, control the medical device to implement an updated target plan on the target subject, wherein the updated target plan is determined based on the adjusted parameter value.

25. The quality assurance system of any one of claims 21-24, wherein the determining the prediction result based on the status information of the medical device and the target plan of the target subject using a quality assurance model includes:
obtaining an image of the target subject; and
determining a three-dimensional dose distribution in the target subject based on the status information of the medical device, the target plan of the target subject, and the image of the target subject using the quality assurance model.

26. The quality assurance system of any one of claims 14-25, wherein the target subject includes a plurality of regions of interest (ROI), the prediction result includes dose distributions corresponding to the plurality of ROIs, and the determining whether the quality assurance test is passed based on the prediction result includes:
obtaining a weight value corresponding to each of the plurality of ROIs; and
determining whether the quality assurance test is passed based on the weight value corresponding to each of the plurality of ROIs and the dose distributions corresponding to the plurality of ROIs.

27. A non-transitory computer-readable medium, comprising at least one set of instructions that, when executed by at least one processor of a computing device, cause the at least one processor to implement a method, comprising:
obtaining status information of a medical device;
obtaining a target plan of a target subject;
determining a prediction result based on the status information of the medical device and the target plan; and
determining whether a quality assurance test is passed based on the prediction result.

28. A quality assurance system, comprising:
a first obtaining module, configured to obtain status information of a medical device;
a second obtaining module, configured to obtain a target plan of a target subject; and
a determination module, configured to determine a prediction result based on the status information of the medical device and the target plan; and determine whether a quality assurance test is passed based on the prediction result.

29. A quality assurance device, configured to implement the quality assurance method of any one of claims 1-13.
